# EUROPEAN PATENT SPECIFICATION

(11) **EP 0 371 805 B1**
(45) Date of publication and mention of the grant of the patent: **26.06.1996**
(21) Application number: 89312488.3
(22) Date of filing: 30.11.1989
(51) Int. Cl.: C07D 211/70, C07D 401/06, C07D 405/04, C07D 409/06, C07D 417/06, A61K 31/445

(54) **Piperidine derivatives and hypotensives containing the same**
Piperidin-Derivate und diese enthaltende Hypotensiva
Dérivés de pipéridine et hypotenseurs les contenant

(30) Priority: 30.11.1988 JP 303461/88; 16.03.1989 JP 64059/89
(43) Date of publication of application: 06.06.1990
(73) Proprietor: AJINOMOTO CO., INC., Tokyo 104 (JP)
(72) Inventor: Syoji, Masataka c/o Central Research Laboratories, Kawasaki-shi Kanagawa-ken (JP); Domoto, Hideki c/o Central Research Laboratories, Kawasaki-shi Kanagawa-ken (JP); Toyota, Kozo c/o Central Research Laboratories, Kawasaki-shi Kanagawa-ken (JP); Yoshimoto, Ryota c/o Central Research Laboratories, Kawasaki-shi Kanagawa-ken (JP); Eguchi, Chikahiko c/o Central Research Lab., Kawasaki-shi Kanagawa-ken (JP); Kamimura, Akira c/o Central Research Laboratories, Kawasaki-shi Kanagawa-ken (JP)
(74) Representative: Ford, Michael Frederick

(56) References cited:
- EP-A- 0 005 607
- EP-A- 0 347 123
- WO-A-89/10369
- DE-A- 2 256 392
- DE-A- 2 423 721
- FR-A- 2 290 202
- GB-A- 1 153 977
- US-A- 3 014 911
- US-A- 4 073 912
- US-A- 4 356 184
- CHEM. PHARM. BULL., vol. 27, no. 9, 1979, pages 2056-2060. Y. NAGAI et al.
- J.MED. CHEM., vol. 17, no. 1. January 1974, pages 57-62, C. KAISER et al.
- J.MED. CHEM., vol. 18, no. 1, January 1975, pages 1-8, F.J. VILLIANI et al.

## Description

The present invention relates to the use of piperidine derivatives as hypotensives and more particularly to the use of certain piperidine derivatives in the preparation of hypotensive medicaments.

It is said that there are about 13,000,000 patients with hypertension in Japan and its frequency of occurrence becomes higher as age is advanced. Further, in the elderly, attention has been paid to hypertension more and more as a risk factor in severe heart and cerebral diseases represented by cardiac infarction or cerebral apoplexy. In recent years, calcium antagonists or angiotensin converting enzyme inhibitors have been widely used as excellent primary selection drugs for treatment of hypertension. But pharmaceutical effects or safety of these hypotensives have recently come into question.

It is therefore required to develop new hypotensives having excellent pharmaceutical effects and safety which can be industrially prepared at low cost in a simple manner.

US-A-4 356 184 describes 1-Piperidinylmethyl benzenamines having anti-allergic and anti-hypertensive activity.

As a result of extensive investigations the present inventors have found that certain piperidine derivatives show an excellent hypotensive activity. The synthesis steps of these derivatives are simple and they can be easily prepared industrially.

Accordingly, the present invention resides in the use in the preparation of a hypotensive medicament of a piperidine derivative of general formula (I) or a pharmaceutically acceptable salt thereof: 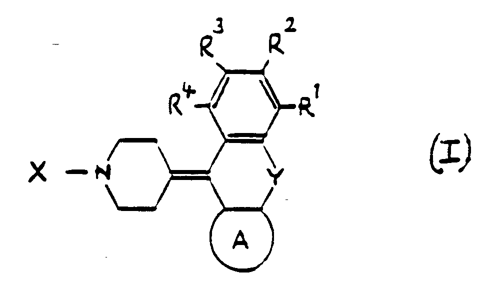
wherein any of R¹, R², R³ and R⁴ may be the same or different from each other and each independently represents a hydrogen atom or a halogen atom, alkyl group having 1 to 4 carbon atoms, hydroxyl group, amino group, cyano group, methoxy group, methylthio group, hydroxymethyl group, carboxyl group, trifluoromethyl group, trifluoromethoxy group, trifluoromethylthio group, trifluoromethylsulfonyl group, hydroxyamino group or nitro group;
Y represents a hetero atom or an optionally substituted alkylene chain, the alkylene chain optionally containing one or more hetero atoms or one or more unsaturated bonds; and
X represents an aralkyl- or aryl-containing group having from 6 to 30 carbon atoms or an alkyl group having from 4 to 30 carbon atoms or a cycloalkyl-containing group, which may optionally have substituent(s) and which may be substituted by hetero atom(s) or hetero atom-containing organic group(s), said alkyl group optionally containing unsaturated bond(s), but wherein X is not a methylbenzene-4-amine group when Y = -CH₂CH₂- or S;
A represents an optionally substituted condensed aromatic or heterocyclic ring;
provided that, if present, the aromatic ring of X or A is benzene, naphthalene, anthracene, pyrrole, furan, thiophene, indole, benzofuran, benzothiophane, pyridine, quinoline isoquinoline, quinolidine, acridine, phenanthridine, pyrazole, imidazole, isoxazole, oxazole, thiazole, isothiazole, pyridazine, pyrazine, purine, puteridine, triazine or benzotriazole.

When used as hypotensives the above-defined piperidine derivatives may be administered by an oral or parenteral route. Those may be determined depending upon age, body weight, condition of the patient and route for administration. A daily dose is generally 0.01 to 2000 mg/kg for oral administration; in the case of parenteral administration, a daily dose is generally 0.01 to 1000 mg/kg. The piperidine derivatives may be formulated into conventional preparation forms, for example, tablets, powders, capsules, solutions, sugar-coated tables or depots, which may be prepared in a conventional manner using conventional techniques. For example, tablets can be obtained by mixing a piperidine derivative with known auxiliary substances, for example, inactive diluents (e.g. lactose, calcium carbonate or calcium phosphate), binders (e.g. gum arabic, corn starch or gelatin), swelling agents (e.g. alginic acid, corn starch or pregelatinated starch), sweeteners (e.g. sucrose or saccharine), flavours (e.g. peppermint, Gaultheria adenothrix oil or cherry), lubricating and wetting agents (e.g. magnesium stearate, talc or carboxymethyl cellulose).

The present invention will now be described in further detail with reference to the examples below.

Unless otherwise indicated, developing conditions for silica gel TLC were chloroform/methanol = 9/1; mass spectrum (MS) was performed in FD mode (m/z) and nuclear magnetic resonance spectrum (NMR) was measured using tetramethylsilane as the internal standard and deuterium chloroform as the solvent.

### Example 1

### 4-(5H-Dibenzo[a,d]cyclohepten-5-ylidene)-1-hexylpiperidine hydrochloride:

A solution of 273 mg (1 mmol) of 4-(5H-dibenzo[a,d]cyclohepten-5-ylidene)-1-hexylpiperidine, 165 mg (1 mmol) of 1-bromohexane, 745 mg (5 mmols) of sodium iodide and 414 mg (3 mmols) of potassium carbonate in 20 ml of methyl isobutyl ketone was stirred and refluxed at 120°C overnight on an oil bath. After the reaction, the mixture was washed by adding 20 ml of water thereto. Then the organic phase was separated and the solvlent was distilled off under reduced pressure. After purifying by silica gel column chromatography (eluent: methanol/chloroform, 1/100 - 1/50), the product was converted into the hydrochloride with an equimolar hydrogen chloride/dioxane solution.
- Amount yielded: 180 mg
- Yield: 46%
- TLC: Rf = 0.68
- MS: 357 (M+)
- NMR: 0.83 (3H, t), 1.2-1.4 (6H, m), 1.7-1.9 (2H, m), 2.31 (2H, dd), 2.53 (2H, d), 2.7-2.8 (2H, m), 3.14 (2H, dd), 3.38 (2H, dd), 3.38 (2H, d), 6.92 (2H, s), 7.2-7.4 (8H, m)
Hereafter procedures were carried out in a manner similar to Example 1.

### Example 2

### 4-(5H-Dibenzo[a,d]cyclohepten-5-ylidene)-1-octylpiperidine hydrochloride:

- Amount yielded: 300 mg
- Yield: 72%
- TLC: Rf = 0.71
- MS: 385 (M+)
- NMR: 0.85 (3H, t), 1.2-1.4 (10H, m), 1.7-2.0 (2H, m), 2.30 (2H, dd), 2.53 (2H, d), 2.7-2.9 (2H, m), 3.13 (2H, dd), 3.38 (2H, d), 6.90 (2H, s), 7.1-7.4 (8H, m)

### Example 3

### 1-Decyl-4-(5H-dibenzo[a,d]cyclohepten-5-ylidene)piperidine hydrochloride:

- Amount yielded: 300 mg
- Yield: 67%
- TLC: Rf = 0.75
- MS: 413 (M+)
- NMR: 0.85 (3H, t), 1.2-1.4 (14H, m), 1.7-1.9 (2H, m), 2.33 (2H, dd), 2.54 (2H, d), 2.7-2.8 (2H, m), 3.15 (2H, dd), 3.39 (2H, d), 6.92 (2H, s), 7.1-7.4 (8H, m)

### Example 4

### 4-(5H-Dibenzo[a,d]cyclohepten-5-ylidene)-1-dodecylpiperidine hydrochloride:

- Amount yielded: 1.10 g
- Yield: 92%
- TLC: Rf = 0.78
- MS: 441 (M+)
- NMR: 0.85 (3H, t), 1.1-1.5 (18H, m), 1.7-1.9 (2H, m), 2.32 (2H, dd), 2.54 (2H, d), 2.7-2.8 (2H, m), 3.12 (2H, dd), 3.36 (2H, d), 6.93 (2H, s), 7.1-7.4 (8H, m)

### Example 5

### 4-(5H-Dibenzo[a,d]cyclohepten-5-ylidene)-1-tetradecylpiperidine hydrochloride:

- Amount yielded: 1.20 g
- Yield: 95%
- TLC: Rf = 0.78
- MS: 469 (M+)
- NMR: 0.82 (3H, t), 1.1-1.5 (22H, m), 1.7-1.9 (2H, m), 2.33 (2H, dd), 2.55 (2H, d), 2.7-2.8 (2H, m), 3.15 (2H, dd), 3.40 (2H, d), 6.92 (2H, s), 7.1-7.4 (8H, m)

### Example 6

### 4-(5H-Dibenzo[a,d]cyclohepten-5-ylidene)-1-hexadecylpiperidine hydrochloride:

- Amount yielded: 1.18g
- Yield: 88%
- TLC: Rf = 0.80
- MS: 497 (M+)
- NMR: 0.80 (3H, t), 1.1-1.6 (26H, m), 1.7-1.9 (2H, m), 2.33 (2H, dd), 2.58 (2H, d), 2.7-2.8 (2H, m), 3.20 (2H, dd), 3.40 (2H, d), 6.88 (2H, s), 7.1-7.4 (8H, m)

### Example 7

### 1-Cyclohexylmethyl-4-(5H-dibenzo[a,d]cyclohepten-5-ylidene)piperidine hydrochloride:

- Amount yielded: 520 mg
- Yield: 51%
- TLC: Rf = 0.75
- MS: 369 (M+)
- NMR: 0.8-2.1 (11H, m), 2.42 (2H, dd), 2.65 (2H, d), 2.78 (2H, d), 3.20 (2H, dd), 3.42 (2H, d), 6.91 (2H, s), 7.1-7.4 (8H, m)

### Example 8

### 1-Cyclohexyl-2-(4-(5H-dibenzo[a,d]cyclohepten-5-ylidene)-1-piperidinyl)ethane hydrochloride:

- Amount yielded: 780 mg
- Yield: 74%
- TLC: Rf = 0.75
- MS: 383 (M+)
- NMR: 0.8-2.1 (13H, m), 2.45 (2H, dd), 2.67 (2H, d), 2.7-2.9 (2H, m), 3.0 (2H, dd), 3.48 (2H, d), 6.94 (2H, s), 7.1-7.4 (8H, m)

### Example 9

### 1-Cyclohexyl-3-(4-(5H-dibenzo[a,d]cyclohepten-5-ylidene)-1-piperidinyl)propane hydrochloride:

- Amount yielded: 1.02 g
- Yield: 94%
- TLC: Rf = 0.77
- MS: 397 (M+)
- NMR: 0.8-2.1 (15H, m), 2.47 (2H, dd), 2.68 (2H, d), 2.7-2.9 (2H, m), 3.0 (2H, dd), 3.49 (2H, d), 6.94 (2H, s), 7.1-7.4 (8H, m)

### Example 10

### 1-Cyclohexyl-4-(4-(5H-dibenzo[a,d]cyclohepten-5-ylidene)-1-piperidinyl)butane hydrochloride:

- Amount yielded: 815 mg
- Yield: 72%
- TLC: Rf = 0.78
- MS: 411 (M+)
- NMR: 0.8-2.1 (17H, m), 2.28 (2H, dd), 2.52 (2H, d), 2.7-2.9 (2H, m), 3.08 (2H, dd), 3.35 (2H, d), 6.92 (2H, s), 7.1-7.4 (8H, m)

### Example 11

### 1-Cyclohexyl-5-(4-(5H-dibenzo[a,d]cyclohepten-5-ylidene)-1-piperidinyl)pentane hydrochloride:

- Amount yielded: 750 mg
- Yield: 65%
- TLC: Rf = 0.80
- MS: 411 (M+)
- NMR: 0.8-2.1 (19H, m), 2.25 (2H, dd), 2.68 (2H, d), 2.7-2.9 (2H, m), 3.12 (2H, dd), 3.38 (2H, d), 6.92 (2H, s), 7.1-7.4 (8H, m)

### Example 12

### 1-Benzyl-4-(5H-dibenzo[a,d]cyclohepten-5-ylidene)piperidine hydrochloride:

- Amount yielded: 320 mg
- Yield: 80%
- TLC: Rf = 0.42
- MS: 363 (M+)
- NMR: 2.28 (2H, dd), 2.52 (2H, d), 3.14 (2H, dd), 3.31 (2H, d), 4.01 (2H, d), 6.90 (2H, s), 7.1-7.6 (13H, m)

### Example 13

### 2-(4-(5H-Dibenzo[a,d]cyclohepten-5-ylidene)-1-piperidinyl)-1-phenylethane hydrochloride:

- Amount yielded: 310 mg
- Yield: 75%
- TLC: Rf = 0.45
- MS: 377 (M+)
- NMR: 2.28 (2H, dd), 2.51 (2H, d), 3.0-3.3 (6H, m), 3.47 (2H, d), 6.90 (2H, s), 7.1-7.4 (13H, m)

### Example 14

### 3-(4-(5H-Dibenzo[a,d]cyclohepten-5-ylidene)-1-piperidinyl)-1-phenylpropane hydrochloride:

- Amount yielded: 330 mg
- Yield: 77%
- TLC: Rf = 0.50
- MS: 391 (M+)
- NMR: 2.1-2.4 (4H, m), 2.51 (2H, d), 2.65 (2H, t), 2.7-2.9 (2H, m), 3.12 (2H, dd), 3.38 (2H, d), 6.90 (2H, s), 7.1-7.4 (13H, m)

### Example 15

### 2-(4-(5H-Dibenzo[a,d]cyclohepten-5-ylidene)-1-piperidinyl)-1-phenoxyethane hydrochloride:

- Amount yielded: 1.95 g
- Yield: 55%
- TLC: Rf = 0.56
- MS: 393 (M+)
- NMR: (fee base)
2.1-2.5 (2H, m), 2.58 (2H, t), 2.6-2.7 (2H, m), 4.05 (2H, t), 6.89 (2H, d), 6.92 (2H, s), 7.1-7.4 (11H, m)

### Example 16

### 3-(4-(5H-Dibenzo[a,d]cyclohepten-5-ylidene)-1-piperidinyl)-1-phenoxypropane hydrochloride:

- Amount yielded: 2.15 g
- Yield: 48%
- TLC: Rf = 0.58
- MS: 407 (M+)
- NMR: (free base)
1.97 (2H, tt), 2.1-2.5 (6H, m), 2.54 (2H, t), 2.6-2.7 (2H, m), 3.97 (2H, dd), 6.86 (2H, d), 6.90 (2H, s), 7.1-7.4 (11H, m)

### Example 17

### 3-(4-(5H-Dibenzo[a,d]cyclohepten-5-ylidene)-1-piperidinyl)-1-phenylthiopropane hydrochloride:

- Amount yielded: 0.85 g
- Yield: 74%
- TLC: Rf = 0.62
- MS: 423 (M+)
- NMR: (free base)
1.73 (2H, tt), 2.0-2.6 (10H, m), 2.80 (2H, t), 6.88 (2H, d), 7.1-7.4 (11H, m)

### Example 18

### 2-(4-(5H-Dibenzo[a,d]cyclohepten-5-ylidene)-1-piperidinyl)-1-(3,4-dimethoxyphenyl)ethane hydrochloride:

- TLC: Rf = 0.78
- MS: 450 (M+)

### Example 19

### 3-(4-(5H-Dibenzo[a,d]cyclohepten-5-ylidene)-1-piperidinyl)propyl-4-fluorophenylsulfoxide hydrochloride:

- TLC: Rf = 0.78
- MS: 457 (M+)

### Example 20

### 3-(4-(5H-Dibenzo[a,d]cyclohepten-5-ylidene)-1-piperidinyl)propyl-4-fluorophenylsulfone hydrochloride:

- TLC: Rf = 0.62
- MS: 473 (M+)

### Example 21

### 4-(5H-Dibenzo[a,d]cyclohepten-5-ylidene)-1-(3-(2-aminophenylthio)propyl)piperidine hydrochloride:

- TLC: Rf = 0.84
- MS: 439 (M+)

### Example 22

### 1-(3-(2-Cinnamoylaminophenylthio)- propyl)-4-(5H-dibenzo[a,d]cyclohepten-5-ylidene)piperidine hydrochloride:

- TLC: Rf = 0.66
- MS: 568 (M+)
- NMR: (free base)
1.74 (2H, tt), 2.0-2.6 (8H, m), 2.80 (2H, t), 6.59 (1H, d), 6.88 (2H, s), 7.0-7.6 (16H, m), 7.75 (1H, d), 8.5 (1H, d), 8.68 (1H, bs)

### Example 23

### 1-Cinnamyl-4-(5H-dibenzo[a,d]cyclohepten-5-ylidene)piperidine hydrochloride:

- TLC: Rf = 0.84
- MS: 389 (M+)
- NMR: (free base)
2.1-2.7 (8H, m), 3.15 (2H, d), 6.25 (1H, td), 6.47 (1H, d), 6.90 (2H, s), 7.1-7.4 (13H, m)

### Example 24

### 2-(4-(5H-Dibenzo[a,d]cyclohepten-5-ylidene)-1-piperidinyl)acetophenone hydrochloride:

- TLC: Rf = 0.71
- MS: 391 (M+)

### Example 25

### 2-(4-(5H-Dibenzo[a,d]cyclohepten-5-ylidene)-1-piperidinyl)-1-hydroxy-1-phenylethane hydrochloride:

- TLC: Rf = 0.36
- MS: 393 (M+)

### Example 26

### 3-(4-(5H-Dibenzo[a,d]cyclohepten-5-ylidene)-1-piperidinyl)propiophenone hydrochloride:

- TLC: Rf = 0.74
- MS: 405 (M+)

### Example 27

### 3-(4-(5H-Dibenzo[a,d]cyclohepten-5-ylidene)-1-piperidinyl)-1-hydroxy-1-phenylpropane hydrochloride:

- TLC: Rf = 0.35
- MS: 407 (M+)

### Example 28

### 2-(4-(5H-Dibenzo[a,d]cyclohepten-5-ylidene)-1-piperidinyl)-4'-fluoroacetophenone hydrochloride:

- TLC: Rf = 0.80
- MS: 409 (M+)

### Example 29

### 2-(4-(5H-Dibenzo[a,d]cyclohepten-5-ylidene)-1-piperidinyl)-1-hydroxy-1-(4-fluorophenyl)ethane hydrochloride:

- TLC: Rf = 0.44
- MS: 411 (M+)

### Example 30

### 3-(4-(5H-Dibenzo[a,d]cyclohepten-5-ylidene)-1-piperidinyl)-4'-fluoropropiophenone hydrochloride:

- TLC: Rf = 0.80
- MS: 423 (M+)

### Example 31

### 3-(4-(5H-Dibenzo[a,d]cyclohepten-5-ylidene)-1-piperidinyl)-1-hydroxy-1-(4-fluorophenyl)propane hydrochloride:

- TLC: Rf = 0.44
- MS: 425 (M+)

### Example 32

### 4-(5H-Dibenzo[a,d]cyclohepten-5-ylidene)-1-(2-fluorobenzyl)piperidine hydrochloride:

- TLC: Rf = 0.75
- MS: 381 (M+)

### Example 33

### 4-(5H-Dibenzo[a,d]cyclohepten-5-ylidene)-1-(3-fluorobenzyl)piperidine hydrochloride:

- TLC: Rf = 0.79
- MS: 381 (M+)

### Example 34

### 4-(5H-Dibenzo[a,d]cyclohepten-5-ylidene)-1-(4-fluorobenzyl)piperidine hydrochloride:

- TLC: Rf = 0.61
- MS: 381 (M+)

### Example 35

### 4-(5H-Dibenzo[a,d]cyclohepten-5-ylidene)-1-(2-trifluoromethylbenzyl)piperidine hydrochloride:

- TLC: Rf = 0.83
- MS: 431 (M+)

### Example 36

### 4-(5H-Dibenzo[a,d]cyclohepten-5-ylidene)-1-(3-trifluoromethylbenzyl)piperidine hydrochloride:

- TLC: Rf = 0.82
- MS: 431 (M+)

### Example 37

### 5-(4-(5H-Dibenzo[a,d]cyclohepten-5-ylidene)-1-(4-trifluoromethylbenzyl)piperidine hydrochloride:

- TLC: Rf = 0.79
- MS: 431 (M+)

### Example 38

### 4-(5H-Dibenzo[a,d]cyclohepten-5-ylidene)-1-(2-methoxybenzyl)piperidine hydrochloride:

- TLC: Rf = 0.61
- MS: 393 (M+)

### Example 39

### 4-(5H-Dibenzo[a,d]cyclohepten-5-ylidene)-1-(3-methoxybenzyl)piperidine hydrochloride:

- TLC: Rf = 0.61
- MS: 393 (M+)

### Example 40

### 4-(5H-Dibenzo[a,d]cyclohepten-5-ylidene)-1-(4-methoxybenzyl)piperidine hydrochloride:

- TLC: Rf = 0.52
- MS: 393 (M+)

### Example 41

### 4-(5H-Dibenzo[a,d]cyclohepten-5-ylidene)-1-pentafluorobenzylpiperidine hydrochloride:

- TLC: Rf = 0.80
- MS: 453 (M+)

### Example 42

### 2-(4-(5H-Dibenzo[a,d]cyclohepten-5-ylidene)-1-piperidinyl)-1-(4-fluorophenyl)ethane hydrochloride

- TLC: Rf = 0.55
- MS: 395 (M+)

### Example 43

### 1-(2-Chlorobenzyl)-4-(5H-dibenzo[a,d]cyclohepten-5-ylidene) piperidine hydrochloride

- TLC: Rf = 0.68
- MS: 397 (M+)

### Example 44

### 1-(3-Chlorobenzyl)-4-(5H-dibenzo[a,d]cyclohepten-5-ylidene) piperidine hydrochloride

- TLC: Rf = 0.58
- MS: 397 (M+)

### Example 45

### 1-(4-Chlorobenzyl)-4-(5H-dibenzo[a,d]cyclohepten-5-ylidene) piperidine hydrochloride

- TLC: Rf = 0.58
- MS: 397 (M+)

### Example 46

### 4-(5H-Dibenzo[a,d]cyclohepten-5-ylidene)-1-(2-methylbenzyl) piperidine hydrochloride

- TLC: Rf = 0.74
- MS: 377 (M+)

### Example 47

### 4-(5H-Dibenzo[a,d]cyclohepten-5-ylidene)-1-(3-methylbenzyl) piperidine hydrochloride

- TLC: Rf = 0.71
- MS: 377 (M+)

### Example 48

### 4-(5H-Dibenzo[a,d]cyclohepten-5-ylidene)-1-(4-methylbenzyl) piperidine hydrochloride

- TLC: Rf = 0.65
- MS: 377 (M+)

### Example 49

### 4-(5H-Dibenzo[a,d]cyclohepten-5-ylidene)-1-heptylpiperidine hydrochloride

- TLC: Rf = 0.70
- MS: 371 (M+)

### Example 50

### 4-(5H-Dibenzo[a,d]cyclohepten-5-ylidene)-1-nonylpiperidine hydrochloride

- TLC: Rf = 0.75
- MS: 399 (M+)

### Example 51

### 4-(5H-Dibenzo[a,d]cyclohepten-5-ylidene)-1-undecylpiperidine hydrochloride

- TLC: Rf = 0.75
- MS: 427 (M+)

### Example 52

### 4-(5H-Dibenzo[a,d]cyclohepten-5-ylidene)-1-tridecylpiperidine hydrochloride

- TLC: Rf = 0.78
- MS: 455 (M+)

### Example 53

### 1-(2-Aminobenzyl)-4-(5H-dibenzo[a,d]cyclohepten-5-ylidene) piperidine dihydrochloride

- TLC: Rf = 0.38
- MS: 392 (M+)

### Example 54

### 1-(3-Aminobenzyl)-4-(5H-dibenzo[a,d]cyclohepten-5-ylidene) piperidine dihydrochloride

- TLC: Rf = 0.23
- MS: 392 (M+)

### Example 55

### 1-(4-Aminobenzyl)-4-(5H-dibenzo[a,d]cyclohepten-5-ylidene) piperidine dihydrochloride

- TLC: Rf = 0.26
- MS: 392 (M+)

### Example 56

### 4-(5H-Dibenzo[a,d]cyclohepten-5-ylidene)-1-(2-nitrobenzyl) piperidine hydrochloride

- TLC: Rf = 0.85
- MS: 422 (M+)

### Example 57

### 4-(5H-Dibenzo[a,d]cyclohepten-5-ylidene)-1-(3-nitrobenzyl) piperidine hydrochloride

- TLC: Rf = 0.82
- MS: 422 (M+)

### Example 58

### 4-(5H-Dibenzo[a,d]cyclohepten-5-ylidene)-1-(4-nitrobenzyl) piperidine hydrochloride

- TLC: Rf = 0.83
- MS: 422 (M+)

### Example 59

### 4-(4-(5H-Dibenzo[a,d]cyclohepten-5-ylidene)-1-piperidinyl)-1-phenyl-1-butene hydrochloride

- TLC: Rf = 0.51
- MS: 403 (M+)

### Example 60

### 1-Benzyloxy-2-(4-(5H-Dibenzo[a,d]cyclohepten-5-ylidene)-1-piperidinyl)ethane hydrochloride

- TLC: Rf = 0.58
- MS: 407 (M+)

### Test Example 1

As test animals, four male spontaneously hypertensive rats (weighing 400 to 440 g) that were sufficiently adapted for feeding and in which hypertension was confirmed were used.

Physiological saline aqueous solution containing 2.5% Nicolle of sample and 2.5% ethanol was intravenously administered at once in a dose of 1 ml/kg. Blood pressure after the administration was measured by non-blood observation blood pressure measurement method.

The results are shown below.

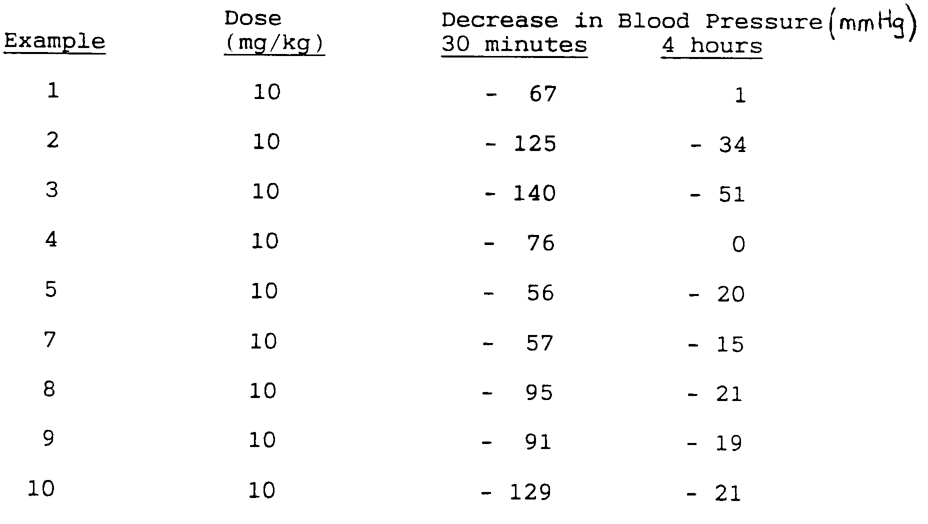

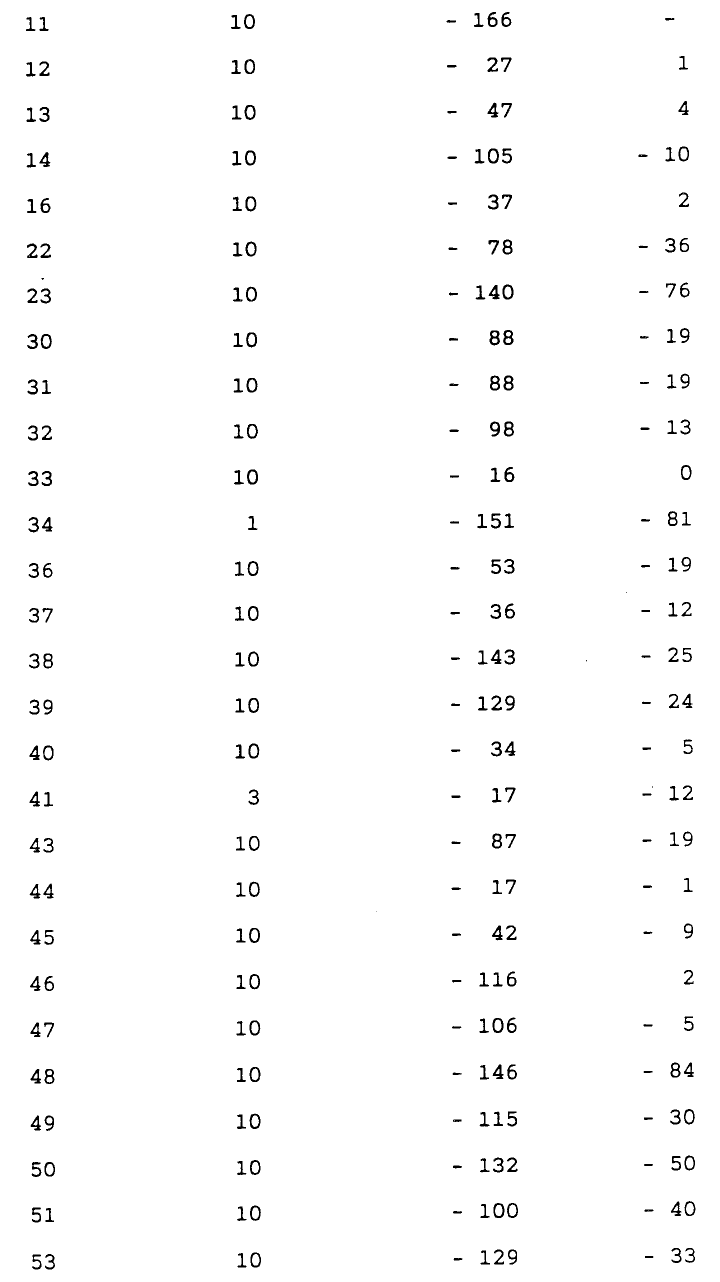

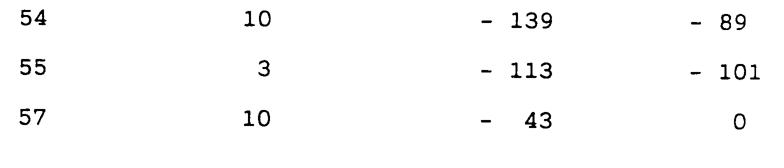

Further Examples 61 to 95 are shown in Table 1 below.

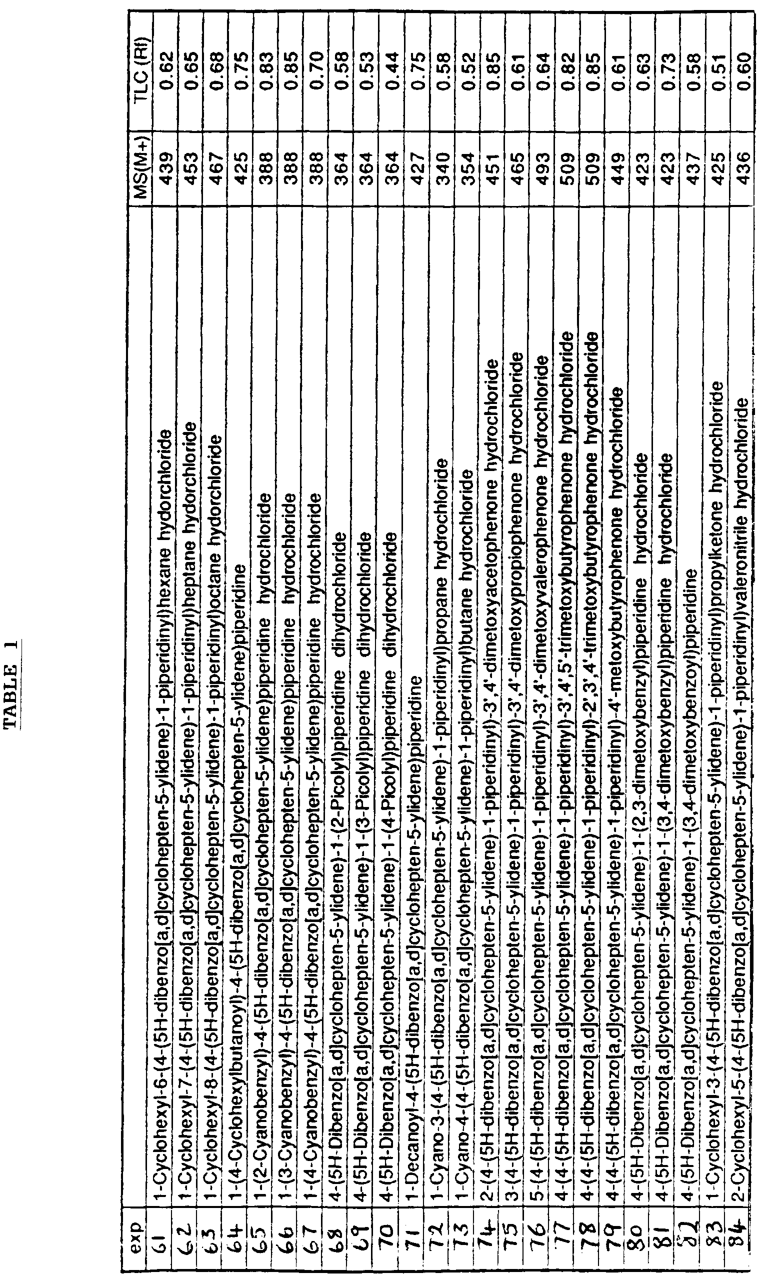

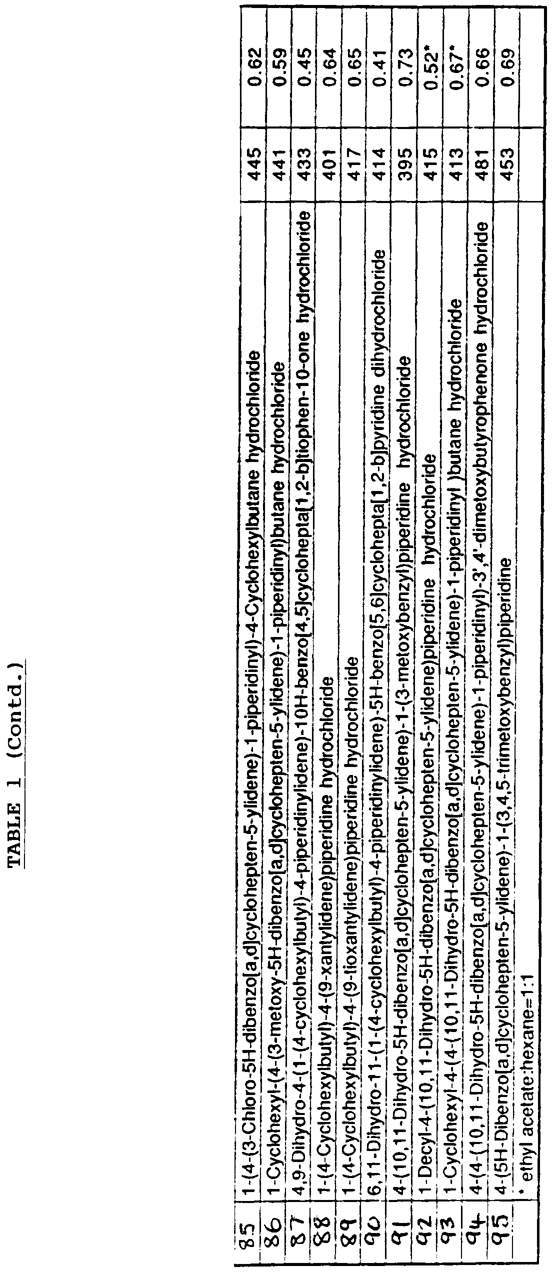

### Test Example 2

Piperidine derivatives of Examples 61 to 95 were tested in the same manner as in Test Example 1. The results are shown below.

| Example | Dose (mg/kg) | Decrease in Blood Pressure (mmHg) | |
|---|---|---|---|
| | | 30 Minutes | 4 Hours |
| 61 | 10 | - 131 | - 105 |
| 64 | 10 | - 32 | - 13 |
| 65 | 10 | - 8 | 7 |
| 66 | 10 | - 27 | - 12 |
| 67 | 10 | - 3 | - 10 |
| 68 | 10 | - 64 | - 12 |
| 69 | 10 | - 62 | - 3 |
| 70 | 10 | - 1 | 4 |
| 71 | 10 | - 91 | - 22 |
| 72 | 10 | - 22 | 12 |
| 73 | 10 | - 45 | 6 |
| 74 | 10 | - 9 | 7 |
| 75 | 10 | - 108 | 0 |
| 76 | 10 | - 130 | - 4 |
| 78 | 10 | - 122 | - 8 |
| 79 | 10 | - 115 | - 25 |
| 80 | 10 | - 98 | - 25 |
| 81 | 10 | - 8 | - 16 |
| 82 | 10 | - 87 | - 1 |
| 92 | 10 | - 66 | - 10 |
| 93 | 10 | - 95 | - 12 |
| 94 | 10 | - 47 | - 9 |
| 95 | 10 | - 94 | - 12 |

Further Examples illustrative of the present invention are Examples 96 to 188 which follow.

### Example 96

### 4-(4-(5H-Dibenzo[a,d]cyclohepten-5-ylidene)-1-piperidinyl)-1-phenylbutane hydrochlcride:

- Amount yielded: 180 mg
- Yield: 41%
- TLC: Rf = 0.50
- MS: 405 (M+)
- NMR: 1.4-1.9 (4H, m), 2.28 (2H, dd), 2.52 (2H, d), 2.61 (2H, t), 2.7-2.8 (2H, m), 3.12 (2H, dd), 3.35 (2H, d), 6.90 (2H, s), 7.1-7.4 (13H, m)

### Example 97

### 5-(4-(5H-Dibenzo[a,d]cyclohepten-5-ylidene)-1-piperidinyl)-1-phenylpentane hydrochloride:

- Amount yielded: 110 mg
- Yield: 24%
- TLC: Rf = 0.55
- MS: 419 (M+)
- NMR: 1.2-1.9 (6H, m), 2.25 (2H, dd), 2.52 (2H, d), 2.60 (2H, t), 2.7-2.8 (2H, m), 3.08 (2H, dd), 3.35 (2H, d), 6.90 (2H, s), 7.1-7.4 (13H, m)

### Example 98

### 6-(4-(5H-Dibenzo[a,d]cyclohepten-5-ylidene)-1-piperidinyl)-1-phenylhexane hydrochloride:

- Amount yielded: 315 mg
- Yield: 67%
- TLC: Rf = 0.56
- MS: 433 (M+)
- NMR: 1.1-1.9 (8H, m), 2.26 (2H, dd), 2.56 (2H, d), 2.61 (2H, t), 2.7-2.8 (2H, m), 3.10 (2H, dd), 3.35 (2H, d), 6.91 (2H, s), 7.1-7.4 (13H, m)

### Example 99

### 7-(4-(5H-Dibenzo[a,d]cyclohepten-5-ylidene)-1-piperidinyl)-1-phenylheptane hydrochloride:

- Amount yielded: 267 mg
- Yield: 55%
- TLC: Rf = 0.56
- MS: 447 (M+)
- NMR: 1.1-1.9 (10H, m), 2.25 (2H, dd), 2.55 (2H, d), 2.65 (2H, t), 2.7-2.8 (2H, m), 3.07 (2H, dd), 3.32 (2H, d), 6.90 (2H, s), 7.1-7.4 (13H, m)

### Example 100

### 4-(4-(5H-Dibenzo[a,d]cyclohepten-5-ylidene)-1-piperidinyl)-1-phenoxybutane hydrochloride:

- Amount yielded: 1.18 g
- Yield: 86%
- TLC: Rf = 0.61
- MS: 421 (M+)
- NMR: (free base)
1.8-2.7 (14H, m), 3.96 (2H, t), 6.87 (2H, d), 6.90 (2H, s), 7.1-7.4 (11H, m)

### Example 101

### 2-(4-(5H-dibenzo[a,d]cyclohepten-5-ylidene)-1-piperidinyl)-1-phenylthioethane hydrochloride:

- Amount yielded: 0.97 g
- Yield: 87%
- TLC: Rf = 0.55
- MS: 409 (M+)

### Example 102

### 4-(4-(5H-Dibenzo[a,d]cyclohepten-5-ylidene)-1-piperidinyl)-1-phenylthiobutane hydrochloride:

- Amount yielded: 0.85 g
- Yield: 72%
- TLC: Rf = 0.62
- MS: 437 (M+)
- NMR: (free base)
1.6-2.6 (14H, m), 2.80 (2H, t), 6.88 (2H, d), 7.1-7.4 (11H, m)

### Example 103

### 4-(5H-Dibenzo[a,d]cyclohepten-5-ylidene)-1-(2-(2-nitrobenzenesulfonyl amino)ethyl)piperidine hydrochloride:

- TLC: Rf = 0.72
- MS: 502 (M+)

### Example 104

### 1-(2-(2-Aminobenzenesulfonyl amino)ethyl)-4-(5H-dibenzo[a,d]cyclohepten-5-ylidene)piperidine hydrochloride:

- TLC: Rf = 0.51
- MS: 472 (M+)

### Example 105

### 4-(5H-Dibenzo[a,d]cyclohepten-5-ylidene)-1-(2-(2-ethoxycarbonylbenzenesulfonyl amino)ethyl)piperidine hydrochloride:

- TLC: Rf = 0.68
- MS: 544 (M+)

### Example 106

### 3-(2-( 4-(5H-Dibenzo[a,d]cyclohepten-5-ylidene)-1-piperidinyl)ethyl)-2,4(1H,3H)quinazolinedione hydrochloride:

- TLC: Rf = 0.85
- MS: 462 (M+)

### Example 107

### 5,6-Benzo-2,4-diaza(2-(4-(5H-dibenzo[a,d]cyclohepten-5-ylidene)-1-piperidinyl)ethyl)tetrahydrothiopyrane-1,1-dioxide hydrochloride:

- TLC: Rf = 0.91
- MS: 498 (M+)

### Example 108

### 5-(4-(5H-Dibenzo[a,d]cyclohepten-5-ylidene)-1-piperidinyl)-2-(3,4-dimethoxyphenyl)-2-isopropylvaleronitrile hydrochloride:

- TLC: Rf = 0.92
- MS: 532 (M+)
- NMR: 0.77 (3H, d), 1.18 (3H, d), 1.6-3.3 (15H, m), 3.86 (3H, s), 3.92 (3H, s), 6.8-7.4 (11H, m)

### Example 109

### 4-(5H-Dibenzo[a,d]cyclohepten-5-ylidene)-1-(2-(benzoylamino)ethyl)piperidine hydrochloride:

- TLC: Rf = 0.84
- MS: 420 (M+)

### Example 110

### 4-(5H-Dibenzo[a,d]cyclohepten-5-ylidene)-1-(2-(phenylcarbamoylamino)ethyl)piperidine hydrochloride:

- TLC: Rf = 0.55
- MS: 435 (M+)

### Example 111

### 5-(4-(5H-Dibenzo[a,d]cyclohepten-5-ylidene)-1-piperidinyl)-2-(3,4,5-trimethoxyphenyl)-2-isopropylvaleronitrile hydrochloride:

- TLC: Rf = 0.80
- MS: 563 (M+)

### Example 112

### 2-(3-(4-(5H-Dibenzo[a,d]cyclohepten-5-ylidene)-1-piperidinyl)propyl)-2-phenyl-1,3-dithiane-1,1,3,3-tetroxide hydrochloride:

- TLC: Rf = 0.48
- MS: 573 (M+)

### Example 113

### 2-(3,4-Dimethoxyphenyl)-2-(3-(4-(5H-dibenzo[a,d]cyclohepten-5-ylidene)-1-piperidinyl)- propyl)-1,3-dithiane-1,1,3,3-tetroxide hydrochloride:

- TLC: Rf = 0.48
- MS: 573 (M+)

### Example 114

### 5-(4-(5H-Dibenzo[a,d]cyclohepten-5-ylidene)-1-piperidinyl)-2-(3,4-dichlorophenyl)-2-isopropylvaleronitrile hydrochloride:

- TLC: Rf = 0.94
- MS: 540 (M+)

### Example 115

### 2-(3-Benzoylphenyl)-5-(4-(5H-dibenzo[a,d]cyclohepten-5-ylidene)-1-piperidinyl)-2-methylvaleronitrile hydrochloride:

- TLC: Rf = 0.88
- MS: 548 (M+)

### Example 116

### 5-(4-(5H-Dibenzo[a,d]cyclohepten-5-ylidene)-1-piperidinyl)-2,2-diphenylvaleronitrile hydrochloride:

- TLC: Rf = 0.74
- MS: 506 (M+)

### Example 117

### 4-(4-(5H-Dibenzo[a,d]cyclohepten-5-ylidene)-1-piperidinyl)-3',4'-dimethoxybutyrophenone hydrochloride:

- TLC: Rf = 0.61
- MS: 479 (M+)

### Example 118

### 6-(4-(5H-Dibenzo[a,d]cyclohepten-5-ylidene)-1-piperidinyl)-2-phenylhexanenitrile hydrochloride:

- TLC: Rf = 0.86
- MS: 430 (M+)

### Example 119

### 6-(4-(5H-Dibenzo[a,d]cyclohepten-5-ylidene)-1-piperidinyl)-2-isopropyl-2-phenylhexanenitrile hydrochloride:

- TLC: Rf = 0.88
- MS: 472 (M+)

### Example 120

### 6-(4-(5H-Dibenzo[a,d]cyclohepten-5-ylidene)-1-piperidinyl)-2-(3,4-dimethoxyphenyl)-2-isopropylhexanenitrile hydrochloride:

- TLC: Rf = 0.81
- MS: 546 (M+)

### Example 121

### 7-(4-(5H-Dibenzo[a,d]cyclohepten-5-ylidene)-1-piperidinyl)-2-phenylheptanenitrile hydrochloride:

- TLC: Rf = 0.84
- MS: 444 (M+)

### Example 122

### 7-(4-(5H-Dibenzo[a,d]cyclohepten-5-ylidene)-1-piperidinyl)-2-isopropyl-2-phenylheptanenitrile hydrochloride:

- TLC: Rf = 0.84
- MS: 486 (M+)

### Example 123

### 7-(4-(5H-Dibenzo[a,d]cyclohepten-5-ylidene)-1-piperidinyl)-2-(3,4-dimethoxyphenyl)-2-isopropylheptanenitrile hydrochloride:

- TLC: Rf = 0.86
- MS: 560 (M+)

### Example 124

### 2-(3-Chloropropyl)-5-(4-(5H-dibenzo[a,d]cyclohepten-5-ylidene)-1-piperidinyl)-2-phenylvaleronitrile hydrochloride:

- TLC: Rf = 0.92
- MS: 506 (M+)

### Example 125

### 5-(4-(5H-Dibenzo[a,d]cyclohepten-5-ylidene)-1-piperidinyl)-2-phenyl-2-phenylthiovaleronitrile hydrochloride:

- TLC: Rf = 0.81
- MS: 538 (M+)

### Example 126

### 5-(4-(5H-Dibenzo[a,d]cyclohepten-5-ylidene)-1-piperidinyl)-2-(3,4-dimethoxyphenyl)-2-phenylthiovaleronitrile hydrochloride:

- TLC: Rf = 0.91
- MS: 598 (M+)

### Example 127

### 5-(4-(5H-Dibenzo[a,d]cyclohepten-5-ylidene)-1-piperidinyl)-2-(1-naphthyl)valeronitrile hydrochloride:

- TLC: Rf = 0.85
- MS: 580 (M+)

### Example 128

### 5-(4-(5H-Dibenzo[a,d]cyclohepten-5-ylidene)-1-piperidinyl)-2-(1-naphthyl)-2-isopropylvaleronitrile hydrochloride:

- TLC: Rf = 0.90
- MS: 522 (M+)

### Example 129

### 5-(4-(5H-Dibenzo[a,d]cyclohepten-5-ylidene)-1-piperidinyl)-2-(2-naphthyl)valeronitrile hydrochloride:

- TLC: Rf = 0.85
- MS: 480 (M+)

### Example 130

### 5-(4-(5H-Dibenzo[a,d]cyclohepten-5-ylidene)-1-piperidinyl)-2-(2-naphthyl)-2-isopropylvaleronitrile hydrochloride:

- TLC: Rf = 0.87
- MS: 522 (M+)

### Example 131

### 5-(4-(5H-Dibenzo[a,d]cyclohepten-5-ylidene)-1-piperidinyl)-2-(3-trifluoromethylphenyl)valeronitrile hydrochloride:

- TLC: Rf = 0.72
- MS: 498 (M+)

### Example 132

### 5-(4-(5H-Dibenzo[a,d]cyclohepten-5-ylidene)-1-piperidinyl)-2-isopropyl-2-(3-trifluoromethylphenyl)valeronitrile hydrochloride:

- TLC: Rf = 0.75
- MS: 540 (M+)

### Example 133

### 8-(4-(5H-Dibenzo[a,d]cyclohepten-5-ylidene)-1-piperidinyl)-2-phenyloctanenitrile hydrochloride:

- TLC: Rf = 0.84
- MS: 472 (M+)

### Example 134

### 8-(4-(5H-Dibenzo[a,d]cyclohepten-5-ylidene)-1-piperidinyl)-2-isopropyl-2-phenyloctanenitrile hydrochloride:

- TLC: Rf = 0.88
- MS: 514 (M+)

### Example 135

### 8-(4-(5H-Dibenzo[a,d]cyclohepten-5-ylidene)-1-piperidinyl)-2-(3,4-dimethoxyphenyl)-2-isopropyloctanenitrile hydrochloride:

- TLC: Rf = 0.82
- MS: 574 (M+)

### Example 136

### 1-(3-(4-(5H-Dibenzo[a,d]cyclohepten-5-ylidene)-1-piperidinyl)propyl)-1-indanecarbonitrile hydrochloride:

- TLC: Rf = 0.90
- MS: 456 (M+)

### Example 137

### 1-(3-(4-(5H-Dibenzo[a,d]cyclohepten-5-ylidene)-1-piperidinyl)propyl)-5,6-dimethoxy-1-indanecarbonitrile hydrochloride:

- TLC: Rf = 0.85
- MS: 516 (M+)

### Example 138

### 5-(4-(5H-Dibenzo[a,d]cyclohepten-5-ylidene)-1-piperidinyl)-2-(1-methylpyrrol-2-yl)valeronitrile hydrochloride:

- TLC: Rf = 0.61
- MS: 433 (M+)

### Example 139

### 5-(4-(5H-Dibenzo[a,d]cyclohepten-5-ylidene)-1-piperidinyl)-2-isopropyl-2-(1-methylpyrrol-2-yl)valeronitrile hydrochlcride:

- TLC: Rf = 0.71
- MS: 475 (M+)

### Example 140

### 5-(4-(5H-Dibenzo[a,d]cyclohepten-5-ylidene)-1-piperidinyl)-2-isopropyl-2-(pyrrol-2-yl)valeronitrile hydrochloride:

- TLC: Rf = 0.55
- MS: 461 (M+)

### Example 141

### 5-(4-(5H-Dibenzo[a,d]cyclohepten-5-ylidene)-1-piperidinyl)-2-(3-(α-hydroxybenzyl)phenyl)-2-methylvaleronitrile hydrochloride:

- TLC: Rf = 0.51
- MS: 550 (M+)

### Example 142

### 2-(3-Benzoylphenyl)-6-(4-(5H-Dibenzo[a,d]cyclohepten-5-ylidene)-1-piperidinyl)-2-methylhexanenitrile hydrochloride:

- TLC: Rf = 0.88
- MS: 562 (M+)

### Example 143

### 6-(4-(5H-Dibenzo[a,d]cyclohepten-5-ylidene)-1-piperidinyl)-2-(3-(α-hydroxybenzyl)phenyl)-2-methylhexanenitrile hydrochloride:

- TLC: Rf = 0.52
- MS: 564 (M+)

### Example 144

### 2-(3-Benzoylphenyl)-7-(4-(5H-Dibenzo[a,d]cyclohepten-5-ylidene)-1-piperidinyl)-2-methylheptanenitrile hydrochloride:

- TLC: Rf = 0.91
- MS: 576 (M+)

### Example 145

### 7-(4-(5H-Dibenzo[a,d]cyclohepten-5-ylidene)-1-piperidinyl)-2-(3-(α-hydroxybenzyl)phenyl)-2-methylheptanenitrile hydrochloride:

- TLC: Rf = 0.52
- MS: 578 (M+)

### Example 146

### 2-(3-Benzoylphenyl)-8-(4-(5H-Dibenzo[a,d]cyclohepten-5-ylidene)-1-piperidinyl)-2-methyloctanenitrile hydrochloride:

- TLC: Rf = 0.90
- MS: 590 (M+)

### Example 147

### 8-(4-(5H-Dibenzo[a,d]cyclohepten-5-ylidene)-1-piperidinyl)-2-(3-(α-hydroxybenzyl)phenyl)-2-methyloctanenitrile hydrochloride:

- TLC: Rf = 0.61
- MS: 592 (M+)

### Example 148

### 5-(4-(5H-Dibenzo[a,d]cyclohepten-5-ylidene)-1-piperidinyl)-2-methyl-2-phenylvaleronitrile hydrochloride:

- TLC: Rf = 0.91
- MS: 544 (M+)

### Example 149

### 5-(4-(5H-Dibenzo[a,d]cyclohepten-5-ylidene)-1-piperidinyl)-2-(3,4-dimethoxyphenyl)-2-methylvaleronitrile hydrochloride:

- TLC: Rf = 0.85
- MS: 504 (M+)

### Example 150

### 5-(4-(5H-Dibenzo[a,d]cyclohepten-5-ylidene)-1-piperidinyl)-2-ethyl-2-phenylvaleronitrile hydrochloride:

- TLC: Rf = 0.92
- MS: 458 (M+)

### Example 151

### 5-(4-(5H-Dibenzo[a,d]cyclohepten-5-ylidene)-1-piperidinyl)-2-(3,4-dimethoxyphenyl)-2-ethylvaleronitrile hydrochloride:

- TLC: Rf = 0.90
- MS: 518 (M+)

### Example 152

### 5-(4-(5H-Dibenzo[a,d]cyclohepten-5-ylidene)-1-piperidinyl)-2-propyl-2-phenylvaleronitrile hydrochloride:

- TLC: Rf = 0.93
- MS: 472 (M+)

### Example 153

### 5-(4-(5H-Dibenzo[a,d]cyclohepten-5-ylidene)-1-piperidinyl)-2-(3,4-dimethoxyphenyl)-2-propylvaleronitrile hydrochloride:

- TLC: Rf = 0.91
- MS: 532 (M+)

### Example 154

### 2-Butyl-5-(4-(5H-Dibenzo[a,d]cyclohepten-5-ylidene)-1-piperidinyl)-2-phenylvaleronitrile hydrochloride:

- TLC: Rf = 0.95
- MS: 486 (M+)

### Example 155

### 2-Butyl-5-(4-(5H-Dibenzo[a,d]cyclohepten-5-ylidene)-1-piperidinyl)-2-(3,4-dimethoxyphenyl)valeronitrile hydrochloride:

- TLC: Rf = 0.90
- MS: 546 (M+)

### Example 156

### 5-(4-(5H-Dibenzo[a,d]cyclohepten-5-ylidene)-1-piperidinyl)-2-pentyl-2-phenylvaleronitrile hydrochloride:

- TLC: Rf = 0.95
- MS: 500 (M+)

### Example 157

### 5-(4-(5H-Dibenzo[a,d]cyclohepten-5-ylidene)-1-piperidinyl)-2-(3,4-dimethoxyphenyl)-2-pentylvaleronitrile hydrochloride:

- TLC: Rf = 0.92
- MS: 560 (M+)

### Example 158

### 5-(4-(5H-Dibenzo[a,d]cyclohepten-5-ylidene)-1-piperidinyl)-2-hexyl-2-phenylvaleronitrile hydrochloride:

- TLC: Rf = 0.95
- MS: 514 (M+)

### Example 159

### 5-(4-(5H-Dibenzo[a,d]cyclohepten-5-ylidene)-1-piperidinyl)-2-(3,4-dimethoxyphenyl)-2-hexylvaleronitrile hydrochloride:

- TLC: Rf = 0.92
- MS: 574 (M+)

### Example 160

### 5-(4-(5H-Dibenzo[a,d]cyclohepten-5-ylidene)-1-piperidinyl)-2-heptyl-2-phenylvaleronitrile hydrochloride:

- TLC: Rf = 0.95
- MS: 528 (M+)

### Example 161

### 5-(4-(5H-Dibenzo[a,d]cyclohepten-5-ylidene)-1-piperidinyl)-2-(3,4-dimethoxyphenyl)-2-heptylvaleronitrile hydrochloride:

- TLC: Rf = 0.91
- MS: 588 (M+)

### Example 162

### 5-(4-(5H-Dibenzo[a,d]cyclohepten-5-ylidene)-1-piperidinyl)-2-octyl-2-phenylvaleronitrile hydrochloride:

- TLC: Rf = 0.94
- MS: 542 (M+)

### Example 163

### 5-(4-(5H-Dibenzo[a,d]cyclohepten-5-ylidene)-1-piperidinyl)-2-(3,4-dimethoxyphenyl)-2-octylvaleronitrile hydrochloride:

- TLC: Rf = 0.94
- MS: 602 (M+)

### Example 164

### 5-(4-(5H-Dibenzo[a,d]cyclohepten-5-ylidene)-1-piperidinyl)-2-nonyl-2-phenylvaleronitrile hydrochloride:

- TLC: Rf = 0.95
- MS: 556 (M+)

### Example 165

### 5-(4-(5H-Dibenzo[a,d]cyclohepten-5-ylidene)-1-piperidinyl)-2-(3,4-dimethoxyphenyl)-2-nonylvaleronitrile hydrochloride:

- TLC: Rf = 0.93
- MS: 616 (M+)

### Example 166

### 2-Decyl-5-(4-(5H-Dibenzo[a,d]cyclohepten-5-ylidene)-1-piperidinyl)-2-phenylvaleronitrile hydrochloride:

- TLC: Rf = 0.95
- MS: 570 (M+)

### Example 167

### 2-Decyl-5-(4-(5H-Dibenzo[a,d]cyclohepten-5-ylidene)-1-piperidinyl)-2-(3,4-dimethoxyphenyl)valeronitrile hydrochloride:

- TLC: Rf = 0.94
- MS: 630 (M+)

### Example 168

### 4-(4-(5H-Dibenzo[a,d]cyclohepten-5-ylidene)-1-piperidinyl)butyrophenone hydrochloride:

- TLC: Rf = 0.75
- MS: 419 (M+)

### Example 169

### 4-(4-(5H-Dibenzo[a,d]cyclohepten-5-ylidene)-1-piperidinyl)-1-hydroxy-1-phenylbutane hydrochloride:

- TLC: Rf = 0.39
- MS: 421 (M+)

### Example 170

### 5-(4-(5H-Dibenzo[a,d]cyclohepten-5-ylidene)-1-piperidinyl)valerophenone hydrochloride:

- TLC: Rf = 0.76
- MS: 433 (M+)

### Example 171

### 5-(5H-Dibenzo[a,d]cyclohepten-5-ylidene)-1-piperidinyl)-1-hydroxy-1-phenylpentane hydrochloride:

- TLC: Rf = 0.78
- MS: 447 (M+)

### Example 172

### 4-(4-(5H-Dibenzo[a,d]cyclohepten-5-ylidene)-1-piperidinyl)-1-hydroxy-1-(4-fluorophenyl)butane hydrochloride:

- TLC: Rf = 0.45
- MS: 439 (M+)

### Example 173

### 5-(4-(5H-Dibenzo[a,d]cyclohepten-5-ylidene)-1-piperidinyl)-4'-fluorovalerophenone hydrochloride:

- TLC: Rf = 0.84
- MS: 451 (M+)

### Example 174

### 5-(4-(5H-Dibenzo[a,d]cyclohepten-5-ylidene)-1-piperidinyl)-1-hydroxy-1-(4-fluorophenyl)pentane hydrochloride:

- TLC: Rf = 0.51
- MS: 453 (M+)

### Example 175

### 5-(4-(5H-Dibenzo[a,d]cyclohepten-5-ylidene)-1-piperidinyl)-2-phenylvaleronitrile hydrochloride:

- TLC: Rf = 0.86
- MS: 440 (M+)

### Example 176

### 5-(4-(5H-Dibenzo[a,d]cyclohepten-5-ylidene)-1-piperidinyl)-2-isopropyl-2-phenylvaleronitrile hydrochloride:

- TLC: Rf = 0.82
- MS: 488 (M+)

### Example 177

### 5-(4-(5H-Dibenzo[a,d]cyclohepten-5-ylidene)-1-piperidinyl)-2-(3,4-dimethoxyphenyl)valeronitrile hydrochloride:

- TLC: Rf = 0.75
- MS: 500 (M+)

### Example 178

### 2-(3-(4-(5H-Dibenzo[a,d]cyclohepten-5-ylidene)-1-piperidinyl)propyl)-2-(4-fluorophenyl)-1,3-dioxolane hydrochloride:

- TLC: Rf = 0.68
- MS: 481 (M+)

### Example 179

### 4-(4-(5H-Dibenzo[a,d]cyclohepten-5-ylidene)-1-piperidinyl)-4'-fluorobutyrophenone hydrochloride:

- TLC: Rf = 0.82
- MS: 437 (M+)

### Example 180

### 5-(4-(5H-Dibenzo[a,d]cyclohepten-5-ylidene)-1-piperidinyl)-2-isopropyl-2-(3,4,5-trimethoxyphenyl)valeronitrile hydrochloride:

- TLC: Rf = 0.66
- MS: 562 (M+)

### Example 181

### 5-(4-(5H-Dibenzo[a,d]cyclohepten-5-ylidene)-1-piperidinyl)-2-(3-fluorophenyl)-2-isopropylvaleronitrile hydrochloride

- TLC: Rf = 0.71
- MS: 500 (M+)

### Example 182

### 5-(4-(5H-Dibenzo[a,d]cyclohepten-5-ylidene)-1-piperidinyl)-2-isopropyl-2-(3-methoxyphenyl)valeronitrile hydrochloride

- TLC: Rf = 0.64
- MS: 512 (M+)

### Example 183

### 5-(4-(5H-Dibenzo[a,d]cyclohepten-5-ylidene)-1-piperidinyl)-2-isopropyl-2-(3-methyl phenyl)valeronitrile hydrochloride

- TLC: Rf = 0.68
- MS: 496 (M+)

### Example 184

### 5-(4-(5H-Dibenzo[a,d]cyclohepten-5-ylidene)-1-piperidinyl)-2-isopropyl-2-(2-trifluoromethylphenyl)valeronitrile hydrochloride

- TLC: Rf = 0.78
- MS: 549 (M+)

### Example 185

### 5-(4-(5H-Dibenzo[a,d]cyclohepten-5-ylidene)-1-piperidinyl)-2-isopropyl-2-(4-trifluoromethylphenyl)valeronitrile hydrochloride

- TLC: Rf = 0.77
- MS: 540 (M+)

### Example 186

### 5-(4-(5H-Dibenzo[a,d]cyclohepten-5-ylidene)-1-piperidinyl)-2-ethyl-2-(3-trifluoromethylphenyl)valeronitrile hydrochloride

- TLC: Rf = 0.77
- MS: 526 (M+)

### Example 187

### 2-Butyl-5-(4-(5H-dibenzo[a,d]cyclohepten-5-ylidene)-1-piperidinyl)-2-(3-trifluoromethylphenyl)valeronitrile hydrochloride

- TLC: Rf = 0.80
- MS: 554 (M+)

### Example 188

### 5-(4-(5H-Dibenzo[a,d]cyclohepten-5-ylidene)-1-piperidinyl)-2-hexyl-2-(3-trifluoromethylphenyl)valeronitrile hydrochloride

- TLC: Rf = 0.80
- MS: 582 (M+)

### Test Example 3

Piperidine derivatives of Examples 96 to 188 were tested in the same manner as in Test Example 1. The results are shown below.

| Example | Dose (mg/kg) | Decrease in Blood Pressure (mmHg) | |
|---|---|---|---|
| | | 30 Minutes | 4 Hours |
| 96 | 10 | - 130 | - 136 |
| 97 | 10 | - 114 | - 30 |
| 98 | 10 | - 84 | - 16 |
| 103 | 10 | - 8 | - 7 |
| 106 | 10 | - 52 | 4 |
| 108 | 10 | - 61 | - 11 |
| 114 | 10 | - 66 | - 14 |
| 116 | 10 | - 14 | 1 |
| 117 | 10 | - 147 | - 38 |
| 124 | 10 | - 23 | - 9 |
| 131 | 10 | - 87 | - 21 |
| 132 | 10 | - 121 | - 48 |
| 168 | 10 | - 35 | - 5 |
| 169 | 10 | - 89 | - 14 |
| 172 | 10 | - 126 | - 50 |
| 175 | 10 | - 42 | - 19 |
| 176 | 10 | - 120 | - 62 |
| 177 | 10 | - 55 | - 7 |
| 178 | 10 | - 90 | - 20 |
| 179 | 10 | - 93 | - 14 |
| 180 | 10 | - 87 | - 19 |
| 186 | 10 | - 116 | - 32 |
| 187 | 10 | - 110 | - 47 |
| 188 | 10 | - 81 | - 43 |

From the foregoing results, it is understood that the piperidine derivatives used according to the present invention provide excellent hypotensives. Accordingly, the present invention is extremely useful, particularly in the pharmaceutical industry.

## Claims

1. Use in the preparation of a hypotensive medicament of a piperidine derivative of general formula (I) or a pharmaceutically acceptable salt thereof: 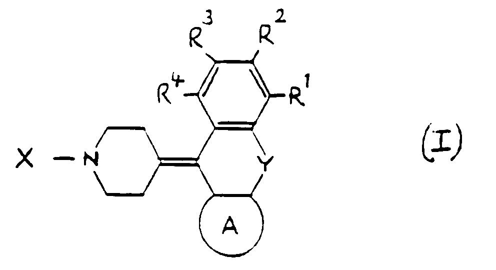 wherein any of R¹, R², R³ and R⁴ may be the same or different from each other and each independently represents a hydrogen atom or a halogen atom, alkyl group having 1 to 4 carbon atoms, hydroxyl group, amino group, cyano group, methoxy group, methylthio group, hydroxymethyl group, carboxyl group, trifluoromethyl group, trifluoromethoxy group, trifluoromethylthio group, trifluoromethylsulfonyl group, hydroxyamino group or nitro group;
Y represents a hetero atom or an optionally substituted alkylene chain, the alkylene chain optionally containing one or more hetero atoms or one or more unsaturated bonds; and
X represents an aralkyl- or aryl-containing group having from 6 to 30 carbon atoms or an alkyl group having from 4 to 30 carbon atoms or a cycloalkyl-containing group, which may optionally have substituent(s) and which may be substituted by hetero atom(s) or hetero atom-containing organic group(s), said alkyl group optionally containing unsaturated bond(s), but wherein X is not a methylbenzene-4-amine group when Y = -CH₂CH₂- or S;
A represents an optionally substituted condensed aromatic or heterocyclic ring;
provided that, if present, the aromatic ring of X or A is benzene, naphthalene, anthracene, pyrrole, furan, thiophene, indole, benzofuran, benzothiophene, pyridine, quinoline, isoquinoline, quinolidine, acridine, phenanthridine, pyrazole, imidazole, isoxazole, oxazole, thiazole, isothiazole, pyridazine, pyrazine, purine, puteridine, triazine or benzotriazole.

2. Use according to Claim 1,
wherein Y in the formula (I) represents an oxygen, sulphur or nitrogen atom or an optionally substituted alkylene chain having 2 or less carbon atoms, said alkylene chain optionally containing oxygen, sulphur and/or nitrogen atom(s) or unsaturated bond(s).

3. Use according to Claim 1, wherein Y represents the group -CH=CH-, -CH₂CH₂-, -O-, -S- or

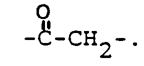

4. Use according to Claim 3, wherein A represents a benzene, thiophene or pyridine ring.

5. Use according to Claim 3 or Claim 4,
wherein R¹, R², R³ and R⁴ each represents a hydrogen atom.

6. Use according to Claim 1, wherein the piperidine derivative is any one of the following:
4-(5H-Dibenzo[a,d]cyclohepten-5-ylidene)-1-hexylpiperidine
4-(5H-Dibenzo[a,d]cyclohepten-5-ylidene)-1-octylpiperidine
1-Decyl-4-(5H-dibenzo[a,d]cyclohepten-5-ylidene)piperidine
4-(5H-Dibenzo[a,d]cyclohepten-5-ylidene)-1-dodecylpiperidine
4-(5H-Dibenzo[a,d]cyclohepten-5-ylidene)-1-tetradecylpiperidine
4-(5H-Dibenzo[a,d]cyclohepten-5-ylidene)-1-hexadecylpiperidine
1-Cyclohexylmethyl-4-(5H-dibenzo[a,d]cyclohepten-5-ylidene)piperidine
1-Cyclohexyl-2-(4-(5H-dibenzo[a,d]cyclohepten-5-ylidene)-1-piperidinyl)ethane
1-Cyclohexyl-3-(4-(5H-dibenzo[a,d]cyclohepten-5-ylidene)-1-piperidinyl)propane
1-Cyclohexyl-4-(4-(5H-dibenzo[a,d]cyclohepten-5-ylidene)-1-piperidinyl)butane
1-Cyclohexyl-5-(4-(5H-dibenzo[a,d]cyclohepten-5-ylidene)-1-piperidinyl)pentane
1-Benzyl-4-(5H-dibenzo[a,d]cyclohepten-5-ylidene)piperidine
2-(4-(5H-Dibenzo[a,d]cyclohepten-5-ylidene)-1-piperidinyl)-1-phenylethane
3-(4-(5H-Dibenzo[a,d]cyclohepten-5-ylidene)-1-piperidinyl)-1-phenylpropane
2-(4-(5H-Dibenzo[a,d]cyclohepten-5-ylidene)-1-piperidinyl)-1-phenoxyethane
3-(4-(5H-Dibenzo[a,d]cyclohepten-5-ylidene)-1-piperidinyl)-1-phenoxypropane
3-(4-(5H-Dibenzo[a,d]cyclohepten-5-ylidene)-1-piperidinyl)-1-phenylthiopropane
2-(4-(5H-Dibenzo[a,d]cyclohepten-5-ylidene)-1-piperidinyl)-1-(3,4-dimethoxyphenyl)ethane
3-(4-(5H-Dibenzo[a,d]cyclohepten-5-ylidene)-1-piperidinyl)propyl-4-fluorophenylsulfoxide
3-(4-(5H-Dibenzo[a,d]cyclohepten-5-ylidene)-1-piperidinyl)propyl-4-fluorophenylsulfone
4-(5H-Dibenzo[a,d]cyclohepten-5-ylidene)-1-(3-(2-aminophenylthio)propyl)piperidine
1-(3-(2-Cinnamoylaminophenylthio)- propyl)-4-(5H-dibenzo[a,d]cyclohepten-5-ylidene)piperidine
1-Cinnamyl-4-(5H-dibenzo[a,d]cyclohepten-5-ylidene)piperidine
2 -(4-(5H-Dibenzo[a,d]cyclohepten-5-ylidene)-1-piperidinyl)-acetophenone
2-(4-(5H-Dibenzo[a,d]cyclohepten-5-ylidene)-1-piperidinyl)-1-hydroxy-1-phenylethane
3-(4-(5H-Dibenzo[a,d]cyclohepten-5-ylidene)-1-piperidinyl)propiophenone
3-(4-(5H-Dibenzo[a,d]cyclohepten-5-ylidene)-1-piperidinyl)-1-hydroxy-1-phenylpropane
2-(4-(5H-Dibenzo[a,d]cyclohepten-5-ylidene)-1-piperidinyl)-4'-fluoroacetophenone
2-(4-(5H-Dibenzo[a,d]cyclohepten-5-ylidene)-1-piperidinyl)-1-hydroxy-1-(4-fluorophenyl)ethane
3-(4-(5H-Dibenzo[a,d]cyclohepten-5-ylidene)-1-piperidinyl)-4'-fluoropropiophenone
3-(4-(5H-Dibenzo[a,d]cyclohepten-5-ylidene)-1-piperidinyl)-1-hydroxy-1-(4-fluorophenyl)propane
4-(5H-Dibenzo[a,d]cyclohepten-5-ylidene)-1-(2-fluorobenzyl)piperidine
4-(5H-Dibenzo[a,d]cyclohepten-5-ylidene)-1-(3-fluorobenzyl)piperidine
4-(5H-Dibenzo[a,d]cyclohepten-5-ylidene)-1-(4-fluorobenzyl)piperidine
4-(5H-Dibenzo[a,d]cyclohepten-5-ylidene)-1-(2-trifluoromethylbenzyl)piperidine
4-(5H-Dibenzo[a,d]cyclohepten-5-ylidene)-1-(3-trifluoromethylbenzyl)piperidine
5-(4-(5H-Dibenzo[a,d]cyclohepten-5-ylidene)-1-(4-trifluoromethylbenzyl) piperidine
4-(5H-Dibenzo[a,d]cyclohepten-5-ylidene)-1-(2-methoxybenzyl)piperidine
4-(5H-Dibenzo[a,d]cyclohepten-5-ylidene)-1-(3-methoxybenzyl)piperidine
4-(5H-Dibenzo[a,d]cyclohepten-5-ylidene)-1-(4-methoxybenzyl)piperidine
4-(5H-Dibenzo[a,d]cyclohepten-5-ylidene)-1-pentafluorobenzylpiperidine
2-(4-(5H-Dibenzo[a,d]cyclohepten-5-ylidene)-1-piperidinyl)-1-(4-fluorophenyl)ethane
1-(2-Chlorobenzyl)-4-(5H-dibenzo[a,d]cyclohepten-5-ylidene) piperidine
1-(3-Chlorobenzyl)-4-(5H-dibenzo[a,d]cyclohepten-5-ylidene) piperidine
1-(4-Chlorobenzyl)-4-(5H-dibenzo[a,d]cyclohepten-5-ylidene) piperidine
4-(5H-Dibenzo[a,d]cyclohepten-5-ylidene)-1-(2-methylbenzyl) piperidine
4-(5H-Dibenzo[a,d]cyclohepten-5-ylidene)-1-(3-methylbenzyl) piperidine
4-(5H-Dibenzo[a,d]cyclohepten-5-ylidene)-1-(4-methylbenzyl) piperidine
4-(5H-Dibenzo[a,d]cyclohepten-5-ylidene)-1-heptylpiperidine
4-(5H-Dibenzo[a,d]cyclohepten-5-ylidene)-1-nonylpiperidine
4-(5H-Dibenzo[a,d]cyclohepten-5-ylidene)-1-undecylpiperidine
4-(5H-Dibenzo[a,d]cyclohepten-5-ylidene)-1-tridecylpiperidine
1-(2-Aminobenzyl)-4-(5H-dibenzo[a,d]cyclohepten-5-ylidene) piperidine
1-(3-Aminobenzyl)-4-(5H-dibenzo[a,d]cyclohepten-5-ylidene) piperidine
1-(4-Aminobenzyl)-4-(5H-dibenzo[a,d]cyclohepten-5-ylidene) piperidine
4-(5H-Dibenzo[a,d]cyclohepten-5-ylidene)-1-(2-nitrobenzyl) piperidine
4-(5H-Dibenzo[a,d]cyclohepten-5-ylidene)-1-(3-nitrobenzyl) piperidine
4-(5H-Dibenzo[a,d]cyclohepten-5-ylidene)-1-(4-nitrobenzyl) piperidine
4-(4-(5H-Dibenzo[a,d]cyclohepten-5-ylidene)-1-piperidinyl) -1-phenyl-1-butene
1-Benzyloxy-2-(4-(5H-Dibenzo[a,d]cyclohepten-5-ylidene)-1-piperidinyl)ethane
1-Cyclohexyl-6-(4-(5H-dibenzo[a,d]cyclohepten-5-ylidene)-1-piperidinyl)hexane
1-Cyclohexyl-7-(4-(5H-dibenzo[a,d]cyclohepten-5-ylidene)-1-piperidinyl)heptane
1-Cyclohexyl-8-(4-(5H-dibenzo[a,d]cyclohepten-5-ylidene)-1-piperidinyl)octane
1-(4-Cyclohexylbutanoyl)-4-(5H-dibenzo[a,d]cyclohepten-5-ylidene)piperidine
1-(2-Cyanobenzyl)-4-(5H-dibenzo[a,d]cyclohepten-5-ylidene)piperidine
1-(3-Cyanobenzyl)-4-(5H-dibenzo[a,d]cyclohepten-5-ylidene)piperidine
1-(4-Cyanobenzyl)-4-(5H-dibenzo[a,d]cyclohepten-5-ylidene)piperidine
4-(5H-Dibenzo[a,d]cyclohepten-5-ylidene)-1-(2-Picolyl)piperidine
4-(5H-Dibenzo[a,d]cyclohepten-5-ylidene)-1-(3-Picolyl)piperidine
4-(5H-Dibenzo[a,d]cyclohepten-5-ylidene)-1-(4-Picolyl)piperidine
1-(Decanoyl)-4-(5H-dibenzo[a,d]cyclohepten-5-ylidene)piperidine
1-Cyano-3-(4-(5H-dibenzo[a,d]cyclohepten-5-ylidene)-1-piperidinyl)propane
1-Cyano-4-(4-(5H-dibenzo[a,d]cyclohepten-5-ylidene)-1-piperidinyl)butane
2-(4-(5H-dibenzo[a,d]cyclohepten-5-ylidene)-1-piperidinyl)-3',4'-dimetoxyacetophenone
3-(4-(5H-dibenzo[a,d]cyclohepten-5-ylidene)-1-piperidinyl)-3',4'-dimetoxyacetophenone
5-(4-(5H-dibenzo[a,d]cyclohepten-5-ylidene)-1-piperidinyl)-3',4'-dimetoxyvalerophenone
4-(4-(5H-dibenzo[a,d]cyclohepten-5-ylidene)-1-piperidinyl)-3',4',5'-trimetoxybutyrophenone
4-(4-(5H-dibenzo[a,d]cyclohepten-5-ylidene)-1-piperidinyl)-2',3',4'-trimetoxybutyrophenone
4-(4-(5H-dibenzo[a,d]cyclohepten-5-ylidene)-1-piperidinyl)-4'-metoxybutyrophenone
4-(5H-Dibenzo[a,d]cyclohepten-5-ylidene)-1-(2,3-dimetoxybenzyl)piperidine
4-(5H-Dibenzo[a,d]cyclohepten-5-ylidene)-1-(3,4-dimetoxybenzyl)piperidine
4-(5H-Dibenzo[a,d]cyclohepten-5-ylidene)-1-(3,4-dimetoxybenzoyl)piperidine
1-Cyclohexyl-3-(4-(5H-dibenzo[a,d]cyclohepten-5-ylidene)-1-piperidinyl)propylketone
2-Cyclohexyl-5-(4-(5H-dibenzo[a,d]cyclohepten-5-ylidene)-1-piperidinyl)valeronitrile
1-(4-(3-Chloro-5H-dibenzo[a,d]cyclohepten-5-ylidene)-1-piperidinyl)-4-Cyclohexylbutane
1-Cyclohexyl-(4-(3-metoxy-5H-dibenzo[a,d]cyclohepten-5-ylidene)-1-piperidinyl)butane
4,9-Dihydro-4-(1-(4-cyclohexylbutyl)-4-piperidylidene)-10H-benzo[4,5]cyclohepta[1,2-b]
tiophen-10-one 1-(4-Cyclohexylbutyl)-4-(9-xantylidene)piperidine
1-(4-Cyclohexylbutyl)-4-(9-tioxantylidene)piperidine
6,11-Dihydro-11-(1-(4-cyclohexylbutyl)-4-piperidinylidene)-5H-benzo[5,6]cyclohepta[1,2-b]
pyridine 4-(10,11-Dihydro-5H-dibenzo[a,d]cyclohepten-5-ylidene)-1-(3-metoxybenzyl)piperidine
1-Decyl-4-(10,11-Dihydro-5H-dibenzo[a,d]cyclohepten-5-ylidene)piperidine
1-Cyclohexyl-4-(4-(10,11-Dihydro-5H-dibenzo[a,d]cyclohepten-5-ylidene)-1-piperidinyl )
butane
4-(4-(10,11-Dihydro-5H-dibenzo[a,d]cyclohepten-5-ylidene)-1-pipendinyl)-3',4'-dimetoxybutyrophenone
4-(5H-Dibenzo[a,d]cyclohepten-5-ylidene)-1-(3,4,5-trimetoxybenzyl)piperidine or a hydrochloride salt thereof;
or any one of the following compounds or a free base form thereof:
4-(4-(5H-Dibenzo[a,d]cyclohepten-5-ylidene)-1-piperidinyl)-1-phenylbutane hydrochloride;
5-(4-(5H-Dibenzo[a,d]cyclohepten-5-ylidene)-1-piperidinyl)-1-phenylpentane hydrochloride;
6-(4-(5H-Dibenzo[a,d]cyclohepten-5-ylidene)-1-piperidinyl)-1-phenylhexane hydrochloride;
7-(4-(5H-Dibenzo[a,d]cyclohepten-5-ylidene)-1-piperidinyl)-1-phenylheptane hydrochloride;
4-(4-(5H-Dibenzo[a,d]cyclohepten-5-ylidene)-1-piperidinyl)-1-phenoxybutane hydrochloride;
2-(4-(5H-dibenzo[a,d]cyclohepten-5-ylidene)-1-piperidinyl)-1-phenylthioethane hydrochloride;
4-(4-(5H-Dibenzo[a,d]cyclohepten-5-ylidene)-1-piperidinyl)-1-phenylthiobutane hydrochloride;
4-(5H-Dibenzo[a,d]cyclohepten-5-ylidene)-1-(2-(2-nitrobenzenesulfonyl amino)ethyl)piperidine hydrochloride;
1-(2-(2-Aminobenzenesulfonyl amino)ethyl)-4-(5H-dibenzo[a,d]cyclohepten-5-ylidene)piperidine hydrochloride;
4-(5H-Dibenzo[a,d]cyclohepten-5-ylidene)-1-(2-(2-ethoxycarbonylbenzenesulfonyl amino)ethyl)piperidine hydrochloride;
3-(2-( 4-(5H-Dibenzo[a,d]cyclohepten-5-ylidene)-1-piperidinyl)ethyl)-2,4(1H,3H)quinazolinedione hydrochloride;
5,6-Benzo-2,4-diaza(2-(4-(5H-dibenzo[a,d]cyclohepten-5-ylidene)-1-piperidinyl)ethyl)tetrahydrothiopyrane-1,1-dioxide hydrochloride;
5-(4-(5H-Dibenzo[a,d]cyclohepten-5-ylidene)-1-piperidinyl)-2-(3,4-dimethoxyphenyl)-2-isopropylvaleronitrile hydrochloride;
4-(5H-Dibenzo[a,d]cyclohepten-5-ylidene)-1-(2-(benzoylamino)ethyl)piperidine hydrochloride;
4-(5H-Dibenzo[a,d]cyclohepten-5-ylidene)-1-(2-(phenylcarbamoylamino)ethyl)piperidine hydrochloride;
5-(4-(5H-Dibenzo[a,d]cyclohepten-5-ylidene)-1-piperidinyl)-2-(3,4,5-trimethoxyphenyl)-2-isopropylvaleronitrile hydrochloride;
2-(3-(4-(5H-Dibenzo[a,d]cyclohepten-5-ylidene)-1-piperidinyl)propyl)-2-phenyl-1,3-dithiane-1,1,3,3-tetroxide hydrochloride;
2-(3,4-Dimethoxyphenyl)-2-(3-(4-(5H-dibenzo[a,d]cyclohepten-5-ylidene)-1-piperidinyl)- propyl)-1,3-dithiane-1,1,3,3-tetroxide hydrochloride;
5-(4-(5H-Dibenzo[a,d]cyclohepten-5-ylidene)-1-piperidinyl)-2-(3,4-dichlorophenyl)-2-isopropylvaleronitrile hydrochloride;
2-(3-Benzoylphenyl)-5-(4-(5H-dibenzo[a,d]cyclohepten-5-ylidene)-1-piperidinyl)-2-methylvaleronitrile hydrochloride;
5-(4-(5H-Dibenzo[a,d]cyclohepten-5-ylidene)-1-piperidinyl)-2,2-diphenylvaleronitrile hydrochloride;
4-(4-(5H-Dibenzo[a,d]cyclohepten-5-ylidene)-1-piperidinyl)-3',4'-dim ethoxybutyrophenone hydrochloride;
6-(4-(5H-Dibenzo[a,d]cyclohepten-5-ylidene)-1-piperidinyl)-2-phenylhexanenitrile hydrochloride;
6-(4-(5H-Dibenzo[a,d]cyclohepten-5-ylidene)-1-piperidinyl)-2-isopropyl-2-phenylhexanenitrile hydrochloride;
6-(4-(5H-Dibenzo[a,d]cyclohepten-5-ylidene)-1-piperidinyl)-2-(3,4-dimethoxyphenyl)-2-isopropylhexanenitrile hydrochloride;
7-(4-(5H-Dibenzo[a,d]cyclohepten-5-ylidene)-1-piperidinyl)-2-phenylheptanenitrile hydrochloride;
7-(4-(5H-Dibenzo[a,d]cyclohepten-5-ylidene)-1-piperidinyl)-2-isopropyl-2-phenylheptanenitrile hydrochloride;
7-(4-(5H-Dibenzo[a,d]cyclohepten-5-ylidene)-1-piperidinyl)-2-(3,4-dimethoxyphenyl)-2-isopropylheptanenitrile hydrochloride;
2-(3-Chloropropyl)-5-(4-(5H-dibenzo[a,d]cyclohepten-5-ylidene)-1-piperidinyl)-2-phenylvaleronitrile hydrochloride;
5-(4-(5H-Dibenzo[a,d]cyclohepten-5-ylidene)-1-piperidinyl)-2-phenyl-2-phenylthiovaleronitrile hydrochloride;
5-(4-(5H-Dibenzo[a,d]cyclohepten-5-ylidene)-1-piperidinyl)-2-(3,4-dimethoxyphenyl)-2-phenylthiovaleronitrile hydrochloride;
5-(4-(5H-Dibenzo[a,d]cyclohepten-5-ylidene)-1-piperidinyl)-2-(1-naphthyl)valeronitrile hydrochloride;
5-(4-(5H-Dibenzo[a,d]cyclohepten-5-ylidene)-1-piperidinyl)-2-(1-naphthyl)-2-isopropylvaleronitrile hydrochloride;
5-(4-(5H-Dibenzo[a,d]cyclohepten-5-ylidene)-1-piperidinyl)-2-(2-naphthyl)valeronitrile hydrochloride;
5-(4-(5H-Dibenzo[a,d]cyclohepten-5-ylidene)-1-piperidinyl)-2-(2-naphthyl)-2-isopropylvaleronitrile hydrochloride;
5-(4-(5H-Dibenzo[a,d]cyclohepten-5-ylidene)-1-piperidinyl)-2-(3-trifluoromethylphenyl)valeronitrile hydrochloride;
5-(4-(5H-Dibenzo[a,d]cyclohepten-5-ylidene)-1-piperidinyl)-2-isopropyl-2-(3-trifluoromethylphenyl)valeronitrile hydrochloride;
8-(4-(5H-Dibenzo[a,d]cyclohepten-5-ylidene)-1-piperidinyl)-2-phenyloctanenitrile hydrochloride;
8-(4-(5H-Dibenzo[a,d]cyclohepten-5-ylidene)-1-piperidinyl)-2-isopropyl-2-phenyloctanenitrile hydrochloride;
8-(4-(5H-Dibenzo[a,d]cyclohepten-5-ylidene)-1-piperidinyl)-2-(3,4-dimethoxyphenyl)-2-isopropyloctanenitrile hydrochloride;
1-(3-(4-(5H-Dibenzo[a,d]cyclohepten-5-ylidene)-1-piperidinyl)propyl)-1-indanecarbonitrile hydrochloride;
1-(3-(4-(5H-Dibenzo[a,d]cyclohepten-5-ylidene)-1-piperidinyl)propyl)-5,6-dimethoxy-1- indanecarbonitrile hydrochloride;
5-(4-(5H-Dibenzo[a,d]cyclohepten-5-ylidene)-1-piperidinyl)-2-(1-methylpyrrol-2-yl)valeronitrile hydrochloride;
5-(4-(5H-Dibenzo[a,d]cyclohepten-5-ylidene)-1-piperidinyl)-2-isopropyl-2-(1-methylpyrrol-2-yl)valeronitrile hydrochloride;
5-(4-(5H-Dibenzo[a,d]cyclohepten-5-ylidene)-1-piperidinyl)-2-isopropyl-2-(pyrrol-2-yl)valeronitrile hydrochloride;
5-(4-(5H-Dibenzo[a,d]cyclohepten-5-ylidene)-1-piperidinyl)-2-(3-(α-hydroxybenzyl)phenyl)-2-methylvaleronitrile hydrochloride;
2-(3-Benzoylphenyl)-6-(4-(5H-Dibenzo[a,d]cyclohepten-5-ylidene)-1-piperidinyl)-2-methylhexanenitrile hydrochloride;
6-(4-(5H-Dibenzo[a,d]cyclohepten-5-ylidene)-1-piperidinyl)-2-(3-(α-hydroxybenzyl)phenyl)-2-methylhexanenitrile hydrochloride;
2-(3-Benzoylphenyl)-7-(4-(5H-Dibenzo[a,d]cyclohepten-5-ylidene)-1-piperidinyl)-2-methylheptanenitrile hydrochloride;
7-(4-(5H-Dibenzo[a,d]cyclohepten-5-ylidene)-1-piperidinyl)-2-(3-(α-hydroxybenzyl)phenyl)-2-methylheptanenitrile hydrochloride;
2-(3-Benzoylphenyl)-8-(4-(5H-Dibenzo[a,d]cyclohepten-5-ylidene)-1-piperidinyl)-2-methyloctanenitrile hydrochloride;
8-(4-(5H-Dibenzo[a,d]cyclohepten-5-ylidene)-1-piperidinyl)-2-(3-(α-hydroxybenzyl)phenyl)-2-methyloctanenitrile hydrochloride;
5-(4-(5H-Dibenzo[a,d]cyclohepten-5-ylidene)-1-piperidinyl)-2-methyl-2-phenylvaleronitrile hydrochloride;
5-(4-(5H-Dibenzo[a,d]cyclohepten-5-ylidene)-1-piperidinyl)-2-(3,4-dimethoxyphenyl)-2-methylvaleronitrile hydrochloride;
5-(4-(5H-Dibenzo[a,d]cyclohepten-5-ylidene)-1-piperidinyl)-2-ethyl -2-phenylvaleronitrile hydrochloride;
5-(4-(5H-Dibenzo[a,d]cyclohepten-5-ylidene)-1-piperidinyl)-2-(3,4-dimethoxyphenyl)-2-ethylvaleronitrile hydrochloride;
5-(4-(5H-Dibenzo[a,d]cyclohepten-5-ylidene)-1-piperidinyl)-2-propyl-2-phenylvaleronitrile hydrochloride;
5-(4-(5H-Dibenzo[a,d]cyclohepten-5-ylidene)-1-piperidinyl)-2-(3,4-dimethoxyphenyl)-2-propylvaleronitrile hydrochloride;
2-Butyl-5-(4-(5H-Dibenzo[a,d]cyclohepten-5-ylidene)-1-piperidinyl)-2-phenylvaleronitrile hydrochloride;
2-Butyl-5-(4-(5H-Dibenzo[a,d]cyclohepten-5-ylidene)-1-piperidinyl)-2-(3,4-dimethoxyphenyl)valeronitrile hydrochloride;
5-(4-(5H-Dibenzo[a,d]cyclohepten-5-ylidene)-1-piperidinyl)-2-pentyl-2-phenylvaleronitrile hydrochloride;
5-(4-(5H-Dibenzo[a,d]cyclohepten-5-ylidene)-1-piperidinyl)-2-(3,4-dimethoxyphenyl)-2-pentylvaleronitrile hydrochloride;
5-(4-(5H-Dibenzo[a,d]cyclohepten-5-ylidene)-1-piperidinyl)-2-hexyl-2-phenylvaleronitrile hydrochloride;
5-(4-(5H-Dibenzo[a,d]cyclohepten-5-ylidene)-1-piperidinyl)-2-(3,4-dimethoxyphenyl)-2-hexylvaleronitrile hydrochloride;
5-(4-(5H-Dibenzo[a,d]cyclohepten-5-ylidene)-1-piperidinyl)-2-heptyl-2-phenylvaleronitrile hydrochloride;
5-(4-(5H-Dibenzo[a,d]cyclohepten-5-ylidene)-1-piperidinyl)-2-(3,4-dimethoxyphenyl)-2-heptylvaleronitrile hydrochloride;
5-(4-(5H-Dibenzo[a,d]cyclohepten-5-ylidene)-1-piperidinyl)-2-octyl-2-phenylvaleronitrile hydrochloride;
5-(4-(5H-Dibenzo[a,d]cyclohepten-5-ylidene)-1-piperidinyl)-2-(3,4-dimethoxyphenyl)-2-octylvaleronitrile hydrochloride;
5-(4-(5H-Dibenzo[a,d]cyclohepten-5-ylidene)-1-piperidinyl)-2-nonyl-2-phenylvaleronitrile hydrochloride;
5-(4-(5H-Dibenzo[a,d]cyclohepten-5-ylidene)-1-piperidinyl)-2-(3,4-dimethoxyphenyl)-2-nonylvaleronitrile hydrochloride;
2-Decyl-5-(4-(5H-Dibenzo[a,d]cyclohepten-5-ylidene)-1-piperidinyl)-2-phenylvaleronitrile hydrochloride;
2-Decyl-5-(4-(5H-Dibenzo[a,d]cyclohepten-5-ylidene)-1-piperidinyl)-2-(3,4-dimethoxyphenyl)valeronitrile hydrochloride;
4-(4-(5H-Dibenzo[a,d]cyclohepten-5-ylidene)-1-piperidinyl)butyrophenone hydrochloride;
4-(4-(5H-Dibenzo[a,d]cyclohepten-5-ylidene)-1-piperidinyl)-1-hydroxy-1-phenylbutane hydrochloride;
5-(4-(5H-Dibenzo[a,d]cyclohepten-5-ylidene)-1-piperidinyl)valerophenone hydrochloride;
5-(5H-Dibenzo[a,d]cyclohepten-5-ylidene)-1-piperidinyl)-1-hydroxy-1-phenylpentane hydrochloride;
4-(4-(5H-Dibenzo[a,d]cyclohepten-5-ylidene)-1-piperidinyl)-1-hydroxy-1-(4-fluorophenyl)butane hydrochloride;
5-(4-(5H-Dibenzo[a,d]cyclohepten-5-ylidene)-1-piperidinyl)-4'-fluorovalerophenone hydrochloride;
5-(4-(5H-Dibenzo[a,d]cyclohepten-5-ylidene)-1-piperidinyl)-1-hydroxy-1-(4-fluorophenyl)pentane hydrochloride;
5-(4-(5H-Dibenzo[a,d]cyclohepten-5-ylidene)-1-piperidinyl)-2-phenylvaleronitrile hydrochloride;
5-(4-(5H-Dibenzo[a,d]cyclohepten-5-ylidene)-1-piperidinyl)-2-isopropyl-2-phenylvaleronitrile hydrochloride;
5-(4-(5H-Dibenzo[a,d]cyclohepten-5-ylidene)-1-piperidinyl)-2-(3,4-dimethoxyphenyl)valeronitrile hydrochloride;
2-(3-(4-(5H-Dibenzo(a,d]cyclohepten-5-ylidene)-1-piperidinyl)propyl)-2-(4-fluorophenyl)-1,3-dioxolane hydrochloride;
4-(4-(5H-Dibenzo[a,d]cyclohepten-5-ylidene)-1-piperidinyl)-4'-fluorobutyrophenone hydrochloride;
5-(4-(5H-Dibenzo[a,d]cyclohepten-5-ylidene)-1-piperidinyl)-2-isopropyl-2-(3,4,5-trimethoxyphenyl)valeronitrile hydrochloride;
5-(4-(5H-Dibenzo[a,d]cyclohepten-5-ylidene)-1-piperidinyl) -2-(3-fluorophenyl)-2-isopropylvaleronitrile hydrochloride;
5-(4-(5H-Dibenzo[a,d]cyclohepten-5-ylidene)-1-piperidinyl) -2-isopropyl-2-(3-methoxyphenyl)valeronitrile hydrochloride;
5-(4-(5H-Dibenzo[a,d]cyclohepten-5-ylidene)-1-piperidinyl) -2-isopropyl-2-(3-methyl phenyl)valeronitrile hydrochloride;
5-(4-(5H-Dibenzo[a,d]cyclohepten-5-ylidene)-1-piperidinyl) -2-isopropyl-2-(2-trifluoromethylphenyl)valeronitrile hydrochloride;
5-(4-(5H-Dibenzo[a,d]cyclohepten-5-ylidene)-1-piperidinyl) -2-isopropyl-2-(4-trifluoromethylphenyl)valeronitrile hydrochloride;
5-(4-(5H-Dibenzo[a,d]cyclohepten-5-ylidene)-1-piperidinyl) -2-ethyl-2-(3-trifluoromethylphenyl)valeronitrile hydrochloride;
2-Butyl-5-(4-(5H-dibenzo[a,d]cyclohepten-5-ylidene)-1-piperidinyl) -2-(3-trifluoromethylphenyl)valeronitrile hydrochloride;
5-(4-(5H-Dibenzo[a,d]cyclohepten-5-ylidene)-1-piperidinyl)-2-hexyl 2-(3-trifluoromethylphenyl)valeronitrile hydrochloride.

7. Use according to any one of claims 1 to 6, wherein the piperidine derivative is used together with an auxiliary substance, said auxiliary substance being any of an inactive diluent, binder, swelling agent, sweetener, flavour, lubricating agent or wetting agent.

## Patentansprüche

1. Verwendung eines Piperidinderivats oder dessen pharmazeutisch geeigneten Salzes zur Herstellung eines blutdrucksenkenden Arzneimittels, wobei das Piperidinderivat die allgemeine Formel (I) aufweist: 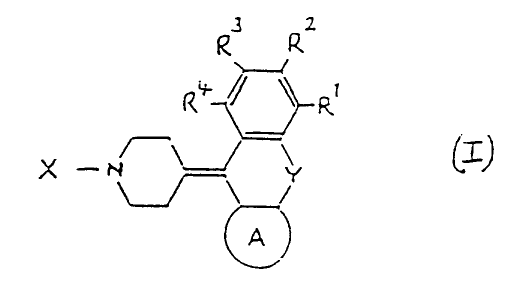 wobei R¹, R², R³ und R⁴ jeweils gleich oder verschieden voneinander sein können und unabhängig voneinander je ein Wasserstoffatom oder ein Halogenatom, eine Alkylgruppe mit 1 bis 4 Kohlenstoffatomen, eine Hydroxylgruppe, eine Aminogruppe, eine Cyangruppe, eine Methoxygruppe, eine Methylthiogruppe, eine Hydroxymethylgruppe, eine Carboxylgruppe, eine Trifluormethylgruppe, eine Trifluormethoxygruppe, eine Trifluormethylthiogruppe, eine Trifluormethylsulfonylgrupe, eine Hydroxylaminogruppe oder eine Nitrogruppe darstellen;
Y ein Heteroatom oder eine gegebenenfalls substituierte Alkylenkette darstellt, wobei die Alkylenkette gegebenenfalls ein oder mehrere Heteroatome oder eine oder mehrere ungesättigte Bindungen enthält; und
X eine Aralkyl- oder Aryl-enthaltende Gruppe mit 6 bis 30 Kohlenstoffatomen oder eine Alkylgruppe mit 4 bis 30 Kohlenstoffatomen oder eine Cycloalkyl-enthaltende Gruppe darstellt, die gegebenenfalls einen oder mehrere Substituenten haben können und die mit einem oder mehreren Heteroatomen oder einer oder mehreren Heteroatom-enthaltenden organischen Gruppen substituiert sein können, wobei die Alkylgruppe gegebenenfalls eine oder mehrere ungesättigte Bindungen enthält, wobei X jedoch keine Methylbenzol-4-aminogruppe ist, wenn Y = -CH₂CH₂- oder S;
A einen gegebenenfalls substituierten kondensierten aromatischen oder heterocyclischen Ring darstellt;
unter der Maßgabe, daß, wenn vorhanden, der aromatische Ring aus X oder A Benzol, Naphthalin, Anthracen, Pyrrol, Furan, Thiophen, Indol, Benzofuran, Benzothiophen, Pyridin, Chinolin, Isochinolin, Chinolidin, Acridin, Phenanthridin, Pyrazol, Imidazol, Isoxazol, Oxazol, Thiazol, Isothiazol, Pyridazin, Pyrazin, Purin, Pteridin, Triazin oder Benzotriazol ist.

2. Verwendung gemäß Anspruch 1, wobei Y in Formel (I) ein Sauerstoff-, Schwefel- oder Stickstoffatom oder eine gegebenenfalls substituierte Alkylenkette mit zwei oder weniger Kohlenstoffatomen darstellt, wobei die Alkylenkette gegebenenfalls ein oder mehrere Sauerstoff-, Schwefel- und/oder Stickstoffatome oder eine oder mehrere ungesättigte Bindungen enthält.

3. Verwendung gemäß Anspruch 1, wobei Y -CH=CH-, -CH₂CH₂-, -O-, -S- oder 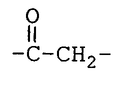 darstellt.

4. Verwendung gemäß Anspruch 3, wobei A einen Benzol-, Thiophen- oder Pyridinring darstellt.

5. Verwendung gemäß Anspruch 3 oder 4, wobei R¹, R², R³ und R⁴ jeweils ein Wasserstoffatom darstellen.

6. Verwendung gemäß Anspruch 1, wobei das Piperidinderivat eines der folgenden ist :
4-(5H-Dibenzo[a,d]cyclohepten-5-yliden)-1-hexylpiperidin
4-(5H-Dibenzo[a,d]cyclohepten-5-yliden)-1-octylpiperidin
1-Decyl-4-(5H-dibenzo[a,d]cyclohepten-5-yliden)piperidin
4-(5H-Dibenzo[a,d]cyclohepten-5-yliden)-1-dodecylpiperidin
4-(5H-Dibenzo[a,d]cyclohepten-5-yliden)-1-tetradecylpiperidin
4-(5H-Dibenzo[a,d]cyclohepten-5-yliden)-1-hexadecylpiperidin
1-Cylohexylmethyl-4-(5H-dibenzo[a,d]cyclohepten-5-yliden)piperidin
1-Cyclohexyl-2-(4-(5H-dibenzo[a,d]cyclohepten-5-yliden)-1-piperidinyl)ethan
1-Cyclohexyl-3-(4-(5H-dibenzo[a,d]cyclohepten-5-yliden)-1-piperidinyl)propan
1-Cyclohexyl-4-(4-(5H-dibenzo[a,d]cyclohepten-5-yliden)-1-piperidinyl)butan
1-Cyclohexyl-5-(4-(5H-dibenzo[a,d]cyclohepten-5-yliden)-1-piperidinyl)pentan
1-Benzyl-4-(5H-dibenzo[a,d]cyclohepten-5-yliden)piperidin
2-(4-(5H-Dibenzo[a,d]cyclohepten-5-yliden)-1-piperidinyl)-1-phenylethan
3-(4-(5H-Dibenzo[a,d]cyclohepten-5-yliden)-1-piperidinyl)-1-phenylpropan
2-(4-(5H-Dibenzo[a,d]cyclohepten-5-yliden)-1-piperidinyl)-1-phenoxyethan
3-(4-(5H-Dibenzo[a,d]cyclohepten-5-yliden)-1-piperidinyl)-1-phenoxypropan
3-(4-(5H-Dibenzo[a,d]cyclohepten-5-yliden)-1-piperidinyl)-1-phenylthiopropan
2-(4-(5H-Dibenzo[a,d]cyclohepten-5-yliden)-1-piperidinyl)-1-(3,4-dimethoxyphenyl)ethan
3-(4-(5H-Dibenzo[a,d]cyclohepten-5-yliden)-1-piperidinyl)propyl-4-fluorphenylsulfoxid
3-(4-(5H-Dibenzo[a,d]cyclohepten-5-yliden)-1-piperidinyl)propyl-4-fluorphenylsulfon
4-(5H-Dibenzo[a,d]cyclohepten-5-yliden)-1-(3-(2-aminophenylthio)propyl)piperidin
1-(3-(2-Cinnamoylaminophenylthio)propyl)-4-(5H-dibenzo[a,d]cyclohepten-5-yliden)piperidin
1-Cinnamyl-4-(5H-dibenzo[a,d]cyclohepten-5-yliden)piperidin
2-(4-(5H-Dibenzo[a,d]cyclohepten-5-yliden)-1-piperidinyl)acetophenon
2-(4-(5H-Dibenzo[a,d]cyclohepten-5-yliden)-1-piperidinyl)-1-hydroxy-1-phenylethan
3-(4-(5H-Dibenzo[a,d]cyclohepten-5-yliden)-1-piperidinyl)propiophenon
3-(4-(5H-Dibenzo[a,d]cyclohepten-5-yliden)-1-piperidinyl)-1-hydroxy-1-phenylpropan
2-(4-(5H-Dibenzo[a,d]cyclohepten-5-yliden)-1-piperidinyl)-4'-fluoracetophenon
2-(4-(5H-Dibenzo[a,d]cyclohepten-5-yliden)-1-piperidinyl)-1-hydroxy-1-(4-fluorphenyl)ethan
3-(4-(5H-Dibenzo[a,d]cyclohepten-5-yliden)-1-piperidinyl)-4'-fluorpropiophenon
3-(4-(5H-Dibenzo[a,d]cyclohepten-5-yliden)-1-piperidinyl)-1-hydroxy-1-(4-fluorphenyl)propan
4-(5H-Dibenzo[a,d]cyclohepten-5-yliden)-1-(2-fluorbenzyl)piperidin
4-(5H-Dibenzo[a,d]cyclohepten-5-yliden)-1-(3-fluorbenzyl)piperidin
4-(5H-Dibenzo[a,d]cyclohepten-5-yliden)-1-(4-fluorbenzyl)piperidin
4-(5H-Dibenzo[a,d]cyclohepten-5-yliden)-1-(2-trifluormethylbenzyl)piperidin
4-(5H-Dibenzo[a,d]cyclohepten-5-yliden)-1-(3-trifluormethylbenzyl)piperidin
5-(4-(5H-Dibenzo[a,d]cyclohepten-5-yliden)-1-(4-trifluormethylbenzyl)piperidin
4-(5H-Dibenzo[a,d]cyclohepten-5-yliden)-1-(2-methoxybenzyl)piperidin
4-(5H-Dibenzo[a,d]cyclohepten-5-yliden)-1-(3-methoxybenzyl)piperidin
4-(5H-Dibenzo[a,d]cyclohepten-5-yliden)-1-(4-methoxybenzyl)piperidin
4-(5H-Dibenzo[a,d]cyclohepten-5-yliden)-1-pentafluorbenzylpiperidin
2-(4-(5H-Dibenzo[a,d]cyclohepten-5-yliden)-1-piperidinyl)-1-(4-fluorphenyl)ethan
1-(2-Chlorbenzyl)-4-(5H-dibenzo[a,d]cyclohepten-5-yliden)piperidin
1-(3-Chlorbenzyl)-4-(5H-dibenzo[a,d]cyclohepten-5-yliden)piperidin
1-(4-Chlorbenzyl)-4-(5H-dibenzo[a,d]cyclohepten-5-yliden)piperidin
4-(5H-Dibenzo[a,d]cyclohepten-5-yliden)-1-(2-methylbenzyl)piperidin
4-(5H-Dibenzo[a,d]cyclohepten-5-yliden)-1-(3-methylbenzyl)piperidin
4-(5H-Dibenzo[a,d]cyclohepten-5-yliden)-1-(4-methylbenzyl)piperidin
4-(5H-Dibenzo[a,d]cyclohepten-5-yliden)-1-heptylpiperidin
4-(5H-Dibenzo[a,d]cyclohepten-5-yliden)-1-nonylpiperidin
4-(5H-Dibenzo[a,d]cyclohepten-5-yliden)-1-undecylpiperidin
4-(5H-Dibenzo[a,d]cyclohepten-5-yliden)-1-tridecylpiperidin
1-(2-Aminobenzyl)-4-(5H-dibenzo[a,d]cyclohepten-5-yliden)piperidin
1-(3-Aminobenzyl)-4-(5H-dibenzo[a,d]cyclohepten-5-yliden)piperidin
1-(4-Aminobenzyl)-4-(5H-dibenzo[a,d]cyclohepten-5-yliden)piperidin
4-(5H-Dibenzo[a,d]cyclohepten-5-yliden)-1-(2-nitrobenzyl)piperidin
4-(5H-Dibenzo[a,d]cyclohepten-5-yliden)-1-(3-nitrobenzyl)piperidin
4-(5H-Dibenzo[a,d]cyclohepten-5-yliden)-1-(4-nitrobenzyl)piperidin
4-(4-(5H-Dibenzo[a,d]cyclohepten-5-yliden)-1-piperidinyl)-1-phenyl-1-buten
1-Benzyloxy-2-(4-(5H-Dibenzo[a,d]cyclohepten-5-yliden)-1-piperidinyl)ethan
1-Cyclohexyl-6-(4-(5H-dibenzo[a,d]cyclohepten-5-yliden)-1-piperidinyl)hexan
1-Cyclohexyl-7-(4-(5H-dibenzo[a,d]cyclohepten-5-yliden)-1-piperidinyl)heptan
1-Cyclohexyl-8-(4-(5H-dibenzo[a,d]cyclohepten-5-yliden)-1-piperidinyl)octan
1-(4-Cyclohexylbutanoyl)-4-(5H-dibenzo[a,d]cyclohepten-5-yliden)piperidin
1-(2-Cyanobenzyl)-4-(5H-dibenzo[a,d]cyclohepten-5-yliden)piperidin
1-(3-Cyanobenzyl)-4-(5H-dibenzo[a,d]cyclohepten-5-yliden)piperidin
1-(4-Cyanobenzyl)-4-(5H-dibenzo[a,d]cyclohepten-5-yliden)piperidin
4-(5H-Dibenzo[a,d]cyclohepten-5-yliden)-1-(2-picolyl)piperidin
4-(5H-Dibenzo[a,d]cyclohepten-5-yliden)-1-(3-picolyl)piperidin
4-(5H-Dibenzo[a,d]cyclohepten-5-yliden)-1-(4-picolyl)piperidin
1-Decanoyl-4-(5H-dibenzo[a,d]cyclohepten-5-yliden)piperidin
1-Cyano-3-(4-(5H-dibenzo[a,d]cyclohepten-5-yliden)-1-piperidinyl)propan
1-Cyano-4-(4-(5H-dibenzo[a,d]cyclohepten-5-yliden)-1-piperidinyl)butan
2-(4-(5H-Dibenzo[a,d]cyclohepten-5-yliden)-1-piperidinyl)-3',4'-dimethoxyaceteophenon
3-(4-(5H-Dibenzo[a,d]cyclohepten-5-yliden)-1-piperidinyl)-3',4'-dimethoxypropiophenon
5-(4-(5H-Dibenzo[a,d]cyclohepten-5-yliden)-1-piperidinyl)-3',4'-dimethoxyvalerophenon
4-(4-(5H-Dibenzo[a,d]cyclohepten-5-yliden)-1-piperidinyl)-3',4',5'-trimethoxybutyrophenon
4-(4-(5H-Dibenzo[a,d]cyclohepten-5-yliden)-1-piperidinyl)-2',3',4'-trimethoxybutyrophenon
4-(4-(5H-Dibenzo[a,d]cyclohepten-5-yliden)-1-piperidinyl)-4'-methoxybutyrophenon
4-(5H-Dibenzo[a,d]cyclohepten-5-yliden)-1-(2,3-dimethoxybenzyl)piperidin
4-(5H-Dibenzo[a,d]cyclohepten-5-yliden)-1-(3,4-dimethoxybenzyl)piperidin
4-(5H-Dibenzo[a,d]cyclohepten-5-yliden)-1-(3,4-dimethoxybenzyl)piperidin
1-Cyclohexyl-3-(4-(5H-dibenzo[a,d]cyclohepten-5-yliden)-1-piperidinyl)propylketon
2-Cyclohexyl-5-(4-(5H-dibenzo[a,d]cyclohepten-5-yliden)-1-piperidinyl)valeronitril
1-(4-(3-Chloro-5H-dibenzo[a,d]cyclohepten-5-yliden)-1-piperidinyl)-4-cyclohexylbutan
1-Cyclohexyl-(4-(3-methoxy-5H-dibenzo[a,d]cyclohepten-5-yliden)-1-piperidinyl)butan
4,9-Dihydro-4-(1-(4-cyclohexylbutyl)-4-piperidinyliden)-10H-benzo[4,5]cyclohepta[1,2-b]thiophen-10-on
1-(4-Cyclohexylbutyl)-4-(9-xanthyliden)piperidin
1-(4-Cyclohexylbutyl)-4-(9-thioxanthyliden)piperidin
6,11-Dihydro-11-(1-(4-cyclohexylbutyl)-4-piperidinyliden)-5H-benzo[5,6]cyclohepta[1,2-b]pyridin
4-(10,11-Dihydro-5H-dibenzo[a,d]cyclohepten-5-yliden)-1-(3-methoxybenzyl)piperidin
1-Decyl-4-(10,11-Dihydro-5H-dibenzo[a,d]cyclohepten-5-yliden)piperidin
1-Cyclohexyl-4-(4-(10,11-Dihydro-5H-dibenzo[a,d]cyclohepten-5-yliden)-1-piperidinyl)butan
4-(4-(10,11-Dihydro-5H-dibenzo[a,d]cyclohepten-5-yliden)-1-piperidinyl)-3',4'-dimethoxybutyrophenon
4-(5H-Dibenzo[a,d]cyclohepten-5-yliden)-1-(3,4,5-trimethoxybenzyl)piperidin
oder deren Hydrochloride;
oder eine der folgenden Verbindungen oder deren freie Base:
4-(4'-(5H-Dibenzo[a,d]cyclohepten-5-yliden)-1-piperidinyl)-1-phenylbutan-hydrochlorid;
5-(4-(5H-Dibenzo[a,d]cyclohepten-5-yliden)-1-piperidinyl)-1-phenylpentan-hydrochlorid;
6-(4-(5H-Dibenzo[a,d]cyclohepten-5-yliden)-1-piperidinyl)-1-phenylhexan-hydrochlorid;
7-(4-(5H-Dibenzo[a,d]cyclohepten-5-yliden)-1-piperidinyl)-1-phenylheptan-hydrochlorid;
4-(4-(5H-Dibenzo[a,d]cyclohepten-5-yliden)-1-piperidinyl)-1-phenoxybutan-hydrochlorid;
2-(4-(5H-Dibenzo[a,d]cyclohepten-5-yliden)-1-piperidinyl)-1-phenylthioethan-hydrochlorid;
4-(4-(5H-Dibenzo[a,d]cyclohepten-5-yliden)-1-piperidinyl-1-phenylthiobutan-hydrochlorid;
4-(5H-Dibenzo[a,d]cyclohepten-5-yliden)-1-(2-(2-nitrobenzolsulfonylamino)ethyl)piperidin-hydrochlorid;
1-(2-(2-Aminobenzolsulfonylamino)ethyl)-4-(5H-dibenzo[a,d]cyclohepten-5-yliden)piperidin-hydrochlorid;
4-(5H-Dibenzo[a,d]cyclohepten-5-yliden)-1-(2-(2-ethoxycarbonylbenzolsulfonylamino)ethyl)piperidin-hydrochlorid;
3-(2-(4-(5H-Dibenzo[a,d]cyclohepten-5-yliden)-1-piperidinyl)ethyl)-2,4(1H,3H)chinazolindion-hydrochlorid;
5,6-Benzo-2,4-diaza(2-(4-(5H-dibenzo[a,d]cyclohepten-5-yliden)-1-piperidinyl)ethyl)tetrahydrothiopyran-1,1-dioxid-hydrochlorid;
5-(4(5H-Dibenzo[a,d]cyclohepten-5-yliden)-1-piperidinyl)-2-(3,4-dimethoxyphenyl)-2-isopropylvaleronitril-hydrochlorid;
4-(5H-Dibenzo[a,d]cyclohepten-5-yliden)-1-(2-(benzoylamino)ethyl)piperidin-hydrochlorid;
4-(5H-Dibenzo[a,d]cyclohepten-5-yliden)-1-(2-(phenylcarbamoylamino)ethyl)piperidin-hydrochlorid;
5-(4-(5H-Dibenzo[a,d]cyclohepten-5-yliden)-1-piperidinyl)-2-(3,4,5-trimethoxyphenyl)-2-isopropylvaleronitril-hydrochlorid;
2-(3-(4-(5H-Dibenzo[a,d]cyclohepten-5-yliden)-1-piperidinyl)propyl)-2-phenyl-1,3-dithian-1,1,3,3-tetroxid-hydrochlorid;
2-(3,4-Dimethoxyphenyl)-2-(3-(4-(5H-dibenzo[a,d]cyclohepten-5-yliden)-1-piperidinyl)propyl)-1,3-dithian-1,1,3,3-tetroxid-hydrochlorid;
5-(4-(5H-Dibenzo[a,d]cyclohepten-5-yliden)-1-piperidinyl)-2-(3,4-dichlorphenyl)-2-isopropylvaleronitril-hydrochlorid;
2-(3-Benzoylphenyl)-5-(4-(5H-dibenzo[a,d]cyclohepten-5-yliden)-1-piperidinyl)-2-methylvaleronitril-hydrochlorid;
5-(4-(5H-Dibenzo[a,d]cyclohepten-5-yliden)-1-piperidinyl)-2,2-diphenylvaleronitril-hydrochlorid;
4-(4-(5H-Dibenzo[a,d]cyclohepten-5-yliden)-1-piperidinyl-3',4'-dimethoxybutyrophenon-hydrochlorid;
6-(4-(5H-Dibenzo[a,d]cyclohepten-5-yliden)-1-piperidinyl)-2-phenylhexannitril-hydrochlorid;
6-(4-(5H-Dibenzo[a,d]cyclohepten-5-yliden)-1-piperidinyl)-2-isopropyl-2-phenylhexannitril-hydrochlorid;
6-(4-(5H-Dibenzo[a,d]cyclohepten-5-yliden)-1-piperidinyl)-2-(3,4-dimethoxyphenyl)-2-isopropylhexannitril-hydrochlorid;
7-(4-(5H-Dibenzo[a,d]cyclohepten-5-yliden)-1-piperidinyl)-2-phenylheptannitril-hydrochlorid;
7-(4-(5H-Dibenzo[a,d]cyclohepten-5-yliden)-1-piperidinyl)-2-isopropyl-2-phenylheptannitril-hydrochlorid;
7-(4-(5H-Dibenzo[a,d]cyclohepten-5-yliden)-1-piperidinyl)-2-(3,4-dimethoxyphenyl)-2-isopropylheptannitril-hydrochlorid;
2-(3-Chlorpropyl)-5-(4-(5H-Dibenzo[a,d]cyclohepten-5-yliden)-1-piperidinyl)-2-phenylvaleronitril-hydrochlorid;
5-(4-(5H-Dibenzo[a,d]cyclohepten-5-yliden)-1-piperidinyl)-2-phenyl-2-phenylthiovaleronitril-hydrochlorid;
5-(4-(5H-Dibenzo[a,d]cyclohepten-5-yliden)-1-piperidinyl)-2-(3,4-dimethoxyphenyl)-2-phenylthiovaleronitril-hydrochlorid;
5-(4-(5H-Dibenzo[a,d]cyclohepten-5-yliden)-1-piperidinyl)-2-(1-naphthyl)valeronitril-hydrochlorid;
5-(4-(5H-Dibenzo[a,d]cyclohepten-5-yliden)-1-piperidinyl)-2-(1-naphthyl)-2-isopropylvaleronitril-hydrochlorid;
5-(4-(5H-Dibenzo[a,d]cyclohepten-5-yliden)-1-piperidinyl)-2-(2-naphthyl)valeronitril-hydrochlorid;
5-(4-(5H-Dibenzo[a,d]cyclohepten-5-yliden)-1-piperidinyl)-2-(2-naphthyl)-2-isopropylvaleronitril-hydrochlorid;
5-(4-(5H-Dibenzo[a,d]cyclohepten-5-yliden)-1-piperidinyl)-2-(3-trifluormethylphenyl)valeronitril-hydrochlorid;
5-(4-(5H-Dibenzo[a,d]cyclohepten-5-yliden)-1-piperidinyl)-2-isopropyl-2-(3-trifluormethylphenyl)valeronitril-hydrochlorid;
8-(4-(5H-Dibenzo[a,d]cyclohepten-5-yliden)-1-piperidinyl)-2-phenyloctannitril-hydrochlorid;
8-(4-(5H-Dibenzo[a,d]cyclohepten-5-yliden)-1-piperidinyl)-2-isopropyl-2-phenyloctannitril-hydrochlorid;
8-(4-(5H-Dibenzo[a,d]cyclohepten-5-yliden)-1-piperidinyl)-2-(3,4-dimethoxyphenyl)-2-isopropyloctannitril-hydrochlorid;
1-(3-(4-(5H-Dibenzo[a,d]cyclohepten-5-yliden)-1-piperidinyl)propyl)-1-indancarbonitril-hydrochlorid;
1-(3-(4-(5H-Dibenzo[a,d]cyclohepten-5-yliden)-1-piperidinyl)propyl)-5,6-dimethoxy-1-indancarbonitril-hydrochlorid;
5-(4-(5H-Dibenzo[a,d]cyclohepten-5-yliden)-1-piperidinyl)-2-(1-methylpyrrol-2-yl)valeronitril-hydrochlorid;
5-(4-(5H-Dibenzo[a,d]cyclohepten-5-yliden)-1-piperidinyl)-2-isopropyl-2-(1-methylpyrrol-2-yl)valeronitril-hydrochlorid;
5-(4-(5H-Dibenzo[a,d]cyclohepten-5-yliden]-1-piperidinyl)-2-isopropyl-2-(pyrrol-2-yl)valeronitril-hydrochlorid;
5-(4-(5H-Dibenzo[a,d]cyclohepten-5-yliden)-1-piperidinyl)-2-(3-(α-hydroxybenzyl)phenyl)-2-methylvaleronitril-hydrochlorid;
2-(3-Benzoylphenyl)-6-(4-(5H-Dibenzo[a,d]cyclohepten-5-yliden)-1-piperidinyl)-2-methylhexannitril-hydrochlorid;
6-(4-(5H-Dibenzo[a,d]cyclohepten-5-yliden)-1-piperidinyl)-2-(3-(α-hydroxybenzyl)phenyl)-2-methylhexannitril-hydrochlorid;
2-(3-Benzoylphenyl)-7-(4-(5H-Dibenzo[a,d]cyclohepten-5-yliden)-1-piperidinyl)-2-methylheptannitril-hydrochlorid;
7-(4-(5H-Dibenzo[a,d]cyclohepten-5-yliden)-1-piperidinyl)-2-(3-(α-hydroxybenzyl)phenyl)-2-methylheptannitril-hydrochlorid;
2-(3-Benzoylphenyl)-8-(4-(5H-Dibenzo[a,d]cyclohepten-5-yliden)-1-piperidinyl)-2-methyloctannitril-hydrochlorid;
8-(4-(5H-Dibenzo[a,d]cyclohepten-5-yliden)-1-piperidinyl)-2-(3-(α-hydroxybenzyl)phenyl)-2-methyloctannitril-hydrochlorid;
5-(4-(5H-Dibenzo[a,d]cyclohepten-5-yliden)-1-piperidinyl)-2-methyl-2-phenylvaleronitril-hydrochlorid;
5-(4-(5H-Dibenzo[a,d]cyclohepten-5-yliden)-1-piperidinyl)-2-(3,4-dimethoxyphenyl)-2-methylvaleronitril-hydrochlorid;
5-(4-(5H-Dibenzo[a,d]cyclohepten-5-yliden)-1-piperidinyl)-2-ethyl-2-phenylvaleronitril-hydrochlorid;
5-(4-(5H-Dibenzo[a,d]cyclohepten-5-yliden)-1-piperidinyl)-2-(3,4-dimethoxyphenyl)-2-ethylvaleronitril-hydrochlorid;
5-(4-(5H-Dibenzo[a,d]cyclohepten-5-yliden)-1-piperidinyl)-2-propyl-2-phenylvaleronitril-hydrochlorid;
5-(4-(5H-Dibenzo[a,d]cyclohepten-5-yliden)-1-piperidinyl)-2-(3,4-dimethoxyphenyl)-2-propylvaleronitril-hydrochlorid;
2-Butyl-5-(4-(5H-Dibenzo[a,d]cyclohepten-5-yliden)-1-piperidinyl)-2-phenylvaleronitril-hydrochlorid;
2-Butyl-5-(4-(5H-Dibenzo[a,d]cyclohepten-5-yliden)-1-piperidinyl)-2-(3,4-dimethoxyphenyl)valeronitril-hydrochlorid;
5-(4-(5H-Dibenzo[a,d]cyclohepten-5-yliden)-1-piperidinyl)-2-pentyl-2-phenylvaleronitril-hydrochlorid;
5-(4-(5H-Dibenzo[a,d]cyclohepten-5-yliden)-1-piperidinyl)-2-(3,4-dimethoxyphenyl)-2-pentylvaleronitril-hydrochlorid;
5-(4-(5H-Dibenzo[a,d]cyclohepten-5-yliden)-1-piperidinyl)-2-hexyl-2-phenylvaleronitril-hydrochlorid;
5-(4-(5H-Dibenzo[a,d]cyclohepten-5-yliden)-1-piperidinyl)-2-(3,4-dimethylphenyl)-2-hexylvaleronitril-hydrochlorid;
5-(4-(5H-Dibenzo[a,d]cyclohepten-5-yliden)-1-piperidinyl)-2-heptyl-2-phenylvaleronitril-hydrochlorid;
5-(4-(5H-Dibenzo[a,d]cyclohepten-5-yliden)-1-piperidinyl)-2-(3,4-dimethoxyphenyl)-2-heptylvaleronitril-hydrochlorid;
5-(4-(5H-Dibenzo[a,d]cyclohepten-5-yliden)-1-piperidinyl)-2-octyl-2-phenylvaleronitril-hydrochlorid;
5-(4-(5H-Dibenzo[a,d]cyclohepten-5-yliden)-1-piperidinyl)-2-(3,4-dimethoxyphenyl)-2-octylvaleronitril-hydrochlorid;
5-(4-(5H-Dibenzo[a,d]cyclohepten-5-yliden)-1-piperidinyl)-2-nonyl-2-phenylvaleronitril-hydrochlorid;
5-(4-(5H-Dibenzo[a,d]cyclohepten-5-yliden)-1-piperidinyl)-2-(3,4-dimethoxyphenyl)-2-nonylvaleronitril-hydrochlorid;
2-Decyl-5-(4-(5H-Dibenzo[a,d]cyclohepten-5-yliden)-1-piperidinyl)-2-phenylvaleronitril-hydrochlorid;
2-Decyl-5-(4-(5H-Dibenzo[a,d]cyclohepten-5-yliden)-1-piperidinyl)-2-(3,4-dimethoxyphenyl)valeronitril-hydrochlorid;
4-(4-(5H-Dibenzo[a,d]cyclohepten-5-yliden)-1-piperidinyl)butyrophenon-hydrochlorid;
4-(4-(5H-Dibenzo[a,d]cyclohepten-5-yliden)-1-piperidinyl)-1-hydroxy-1-phenylbutan-hydrochlorid;
5-(4-(5H-Dibenzo[a,d]cyclohepten-5-yliden)-1-piperidinyl)valerophenon-hydrochlorid;
5-(5H-Dibenzo[a,d]cyclohepten-5-yliden)-1-piperidinyl)-1-hydroxy-1-phenylpentan-hydrochlorid;
4-(4-(5H-Dibenzo[a,d]cyclohepten-5-yliden)-1-piperidinyl)-1-hydroxy-1-(4-fluorphenyl)butan-hydrochlorid;
5-(4-(5H-Dibenzo[a,d]cyclohepten-5-yliden)-1-piperidinyl)-4'-fluorvalerophenon-hydrochlorid;
5-(4-(5H-Dibenzo[a,d]cyclohepten-5-yliden)-1-piperidinyl)-1-hydroxy-1-(4-fluorphenyl)pentan-hydrochlorid;
5-(4-(5H-Dibenzo[a,d]cyclohepten-5-yliden)-1-piperidinyl)-2-phenylvaleronitril-hydrochlorid;
5-(4-(5H-Dibenzo[a,d]cyclohepten-5-yliden)-1-piperidinyl)-2-isopropyl-2-phenylvaleronitril-hydrochlorid;
5-(4-(5H-Dibenzo[a,d]cyclohepten-5-yliden)-1-piperidinyl)-2-(3,4-dimethoxyphenyl)valeronitril-hydrochlorid;
2-(3-(4-(5H-Dibenzo[a,d]cyclohepten-5-yliden)-1-piperidinyl)propyl)-2-(4-fluorphenyl)-1,3-dioxolan-hydrochlorid;
4-(4-(5H-Dibenzo[a,d]cyclohepten-5-yliden)-1-piperidinyl)-4'-fluorbutyrophenon-hydrochlorid;
5-(4-(5H-Dibenzo[a,d]cyclohepten-5-yliden)-1-piperidinyl)-2-isopropyl-2-(3,4,5-trimethoxyphenyl)valeronitril-hydrochlorid;
5-(4-(5H-Dibenzo[a,d]cyclohepten-5-yliden)-1-piperidinyl)-2-(3-fluorphenyl)-2-isopropylvaleronitil-hydrochlorid;
5-(4-(5H-Dibenzo[a,d]cyclohepten-5-yliden)-1-piperidinyl)-2-isopropyl-2-(3-methoxyphenyl)valeronitril-hydrochlorid;
5-(4-(5H-Dibenzo[a,d]cyclohepten-5-yliden)-1-piperidinyl)-2-isopropyl-2-(3-methylphenyl)valeronitril-hydrochlorid;
5-(4-(5H-Dibenzo[a,d]cyclohepten-5-yliden)-1-piperidinyl)-2-isopropyl-2-(2-trifluormethylphenyl)valeronitril-hydrochlorid;
5-(4-(5H-Dibenzo[a,d]cyclohepten-5-yliden)-1-piperidinyl)-2-isopropyl-2-(4-trifluormethylphenyl)valeronitril-hydrochlorid;
5-(4-(5H-Dibenzo[a,d]cyclohepten-5-yliden)-1-piperidinyl)-2-ethyl-2-(3-trifluormethylphenyl)valeronitril-hydrochlorid;
2-Butyl-5-(4-(5H-Dibenzo[a,d]cyclohepten-5-yliden)-1-piperidinyl)-2-(3-trifluormethylphenyl)valeronitril-hydrochlorid;
5-(4-(5H-Dibenzo[a,d]cyclohepten-5-yliden)-1-piperidinyl)-2-hexyl-2-(3-trifluormethylphenyl)valeronitril-hydrochlorid.

7. Verwendung gemäß einem der Ansprüche 1 bis 6, wobei das Piperidinderivat zusammen mit einer Hilfssubstanz verwendet wird, wobei die Hilfssubstanz ein inertes Verdünnungsmittel, ein Bindemittel, ein Quellungsmittel, ein Süßstoff, ein Geschmacksstoff, ein Gleitmittel oder ein Benetzungsmittel ist.

## Revendications

1. Utilisation à la préparation d'un médicament hypotenseur d'un dérivé de pipéridine de formule générale (I) ou un de ses sels pharmaceutiquement acceptables : 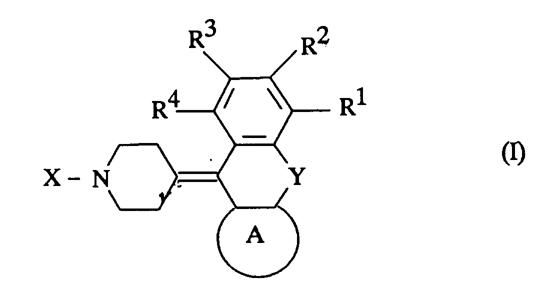 où les groupes R¹, R², R³ et R⁴ peuvent être identiques ou différents l'un de l'autre et chacun représente indépendamment un atome d'hydrogène ou un atome d'halogène, un groupe alkyle ayant 1 à 4 atomes de carbone, un groupe hydroxyle, un groupe amino, un group cyano, un groupe méthoxy, un groupe méthylthio, un groupe hydroxyméthyle, un groupe carboxyle, un groupe trifluorométhyle, un groupe trifluorométhoxy, un groupe difluorométhylthio, un groupe trifluorométhylsulfonyle, un groupe hydroxyamino ou un groupe nitro ;
Y représente un hétéroatome ou une chaîne alkylène substituée éventuellement, la chaîne alkylène contenant éventuellement un ou plusieurs hétéroatomes ou une ou plusieurs liaisons non saturées ; et
X représente un groupe contenant un aralkyle ou un aryle ayant de 6 à 30 atomes de carbone ou un groupe alkyle ayant de 4 à 30 atomes de carbone ou un groupe contenant un cycloalkyle, qui peut éventuellement avoir un substituant ou des substituants et qui peut être substitué par un ou plusieurs hétéroatomes ou un ou plusieurs groupes organiques contenant des hétéroatomes, ledit groupe alkyle contenant éventuellement une ou plusieurs liaisons non saturées, mais où X n'est pas le groupe méthylbenzène-4-amine quand Y = -CH₂CH₂- ou S ;
A représente un cycle aromatique ou hétérocyclique condensé éventuellement substitué ;
à condition que s'il existe, le cycle aromatique de X ou A est le berizène, naphtalène, anthracène, pyrrole, furane, thiophène, indole, benzofurane, benzothiophène, pyridine, quinoléine, isoquinoléine, quinolidine, acridine, phénanthridine, pyrazole, imidazole, isoxazole, oxazole, thiazole, isothiazole, pyridazine, pyazine, purine, putéridine, triazine et benzotriazole.

2. Utilisation selon la revendication 1, où Y dans la formule (I) représente un atome d'oxygène, de soufre ou d'azote ou une chaîne alkylène éventuellement substituée ayant 2 atomes de carbone ou moins, ladite chaîne alkylène contenant éventuellement un ou plusieurs atomes d'oxygène, de soufre et/ou d'azote ou une ou plusieurs liaisons insaturées.

3. Utilisation selon la revendication 1, où Y représente le groupe -CH=CH-, -CH₂CH₂-, -O-, -S- ou

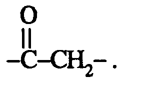

4. Utilisation selon la revendication 3, où A représente un cycle benzène, thiophène ou pyridine.

5. Utilisation selon la revendication 3 ou la revendication 4, où R¹, R², R³ et R⁴ représentent chacun un atome d'hydrogène.

6. Utilisation selon la revendication 1, où le dérivé de pipéridine est l'un quelconque des composés suivants :
4-(5H-dibenzo[a,d]cycloheptène-5-ylidène)-1-hexylpipéridine
4-(5H-dibenzo[a,d]cyclohepten-5-ylidène)-1-octylpipéridine
1-décyl-4-(5H-dibenzo[a,d]cycloheptène-5-ylidène)pipéridine
4-(5H-dibenzo[a,d]cycloheptène-5-ylidène)-1-dodécylpipéridine
4-(5H-dibenzo[a,d]cycloheptène-5-ylidène)-1-tétradécylpipéridine
4-(5H-dibenzo[a,d]cycloheptène-5-ylidène)-1-hexadécylpipéridine
1-cyclohexylméthyl-4-(5H-dibenzo[a,d]cycloheptène-5-ylidène)pipéridine
1-cyclohexyl-2-(4-(5H-dibenzo[a,d]cycloheptène-5-ylidène)-1-pipéridinyl)éthane
1-cyclohexyl-3-(4-(5H-dibenzo[a,b]cycloheptène-5-ylidène)-1-pipéridinyl)propane
1-cyclohexyl-4-(4-(5H-dibenzo[a,d]cycloheptène-5-ylidène)-1-pipéridinyl)butane
1-cyclohexyl-5-(4-(5H-dibenzo[a,d]cycloheptène-5-ylidène)-1-pipéridinyl)pentane
1-benzyl-4-(5H-dibenzo[a,d]cycloheptène-5-ylidène)pipéridine
2-(4-(5H-dibenzo[a,d]cycloheptène-5-ylidène)-1-pipéridinyl)-1-phényléthane
3-(4-(5H-dibenzo[a,d]cycloheptène-5-ylidène)-1-pipéridinyl)-1-phénylpropane
2-(4-(5H-dibenzo[a,d]cycloheptène-5-ylidène)-1-pipéridinyl)-1-phénoxyéthane
3-(4-(5H-dibenzo[a,d]cycloheptène-5-ylidène)-1-pipéridinyl)-1-phénoxypropane
3-(4-(5H-dibenzo[a,d]cycloheptène-5-ylidène)-1-pipéridinyl)-1-phénylthiopropane
2-(4-(5H-dibenzo[a,d]cycloheptène-5-ylidène)-1-pipéridinyl)-1-(3,4-diméthoxyphényl)éthane
3-(4-(5H-dibenzo[a,d]cycloheptène-5-ylidène)-1-pipéridinyl)propyl-4-fluorophénylsulfoxyde
3-(4-(5H-dibenzo[a,d]cycloheptène-5-ylidène)-1-pipéridinyl)propyl-4-fluorophénylsulfone
4-(5H-dibenzo[a,d]cycloheptène-5-ylidène)-1-(3-(2-aminophénylthio)propyl)pipéridine
1-(3-(2-cinnamoylaminophénylthio)-propyl)-4-(5H-dibenzo[a,d]-cycloheptène-5-ylidène)pipéridine
1-cinnamyl-4-(5H-dibenzo[a,d]cycloheptène-5-ylidène)pipéridine
2-(4-(5H-dibenzo[a,d]cycloheptène-5-ylidène)-1-pipéridinyl)-acétophénone
2-(4-(5H-dibenzo[a,d]cycloheptène-5-ylidène)-1-pipéridinyl)-1-hydroxy-1 phényléthane
3-(4-(5H-dibenzo[a,d]cycloheptène-5-ylidène)-1-pipéridinyl)propiophénone
3-(4-(5H-dihenzo[a,d]cycloheptène-5-ylidène)-1-pipéridinyl)-1-hydroxy-1-phénylpropane
2-(4-(5H-dibenzo[a,d]cycloheptène-5-ylidène)-1-pipéridinyl)-4'-fluoroacétophénone
2-(4-(5H-dibenzo[a,d]cycloheptène-5-ylidène)-1-pipéridinyl)-1-hydroxy-1-(4-fluorophényl)éthane
3-(4-(5H-dibenzo[a,d]cycloheptène-5-ylidène)-1-pipéridinyl)-4'-fluoropropiophénone
3-(4-(5H-dibenzo[a,d]cycloheptène-5-ylidène)-1-pipéridinyl)-1-hydroxy-1-(4-fluorophényl)propane
4-(5H-dibenzo[a,d]cycloheptène-5-ylidène)-1-(2-fluorobenzyl)pipéridine
4-(5H-dibenzo[a,d]cycloheptène-5-ylidène)-1-(3-fluorobenzyl)pipéridine
4-(5H-dibenzo[a,d]cycloheptène-5-ylidène)-1-(4-fluorobenzyl)pipéridine
4-(5H-dibenzo[a,d]cycloheptène-5-ylidène)-1-(2-trifluorométhylbenzyl)pipéridine
4-(5H-dibenzo[a,d]cycloheptène-5-ylidène)-1-(3-trifluorométhylbenzyl)pipéridine
5-(4-(5H-dibenzo[a,d]cycloheptène-5-ylidène)-1-(4-trifluorométhylbenzyl)pipéridine
4-(5H-dibenzo[a,d]cycloheptène-5-ylidène)-1-(2-méthoxybenzyl)pipéridine
4-(5H-dibenzo[a,d]cycloheptène-5-ylidène)-1-(3-méthoxybenzyl)pipéridine
4-(5H-dibenzo[a,d]cycloheptène-5-ylidène)-1-(4-méthoxybenzyl)pipéridine
4-(5H-dibenzo[a,d]cycloheptène-5-ylidène)-1-pentafluorobenzyl)pipéridine
2-(4-(5H-dibenzo[a,d]cycloheptène-5-ylidène)-1-pipéridinyl)-1-(4-fluorophényl)éthane
1-(2-chlorobenzyl)-4-(5H-dibenzo[a,d]cycloheptène-5-ylidène)pipéridine
1-(3-chlorobenzyl)-4-(5H-dibenzo[a,d]cycloheptène-5-ylidène)pipéridine
1-(4-chlorobenzyl)-4-(5H-dibenzo[a,d]cycloheptène-5-ylidène)pipéridine
4-(5H-dibenzo[a,d]cycloheptène-5-ylidène)-1-(2-méthylbenzyl)pipéridine
4-(5H-dibenzo[a,d]cycloheptène-5-ylidène)-1-(3-méthylbenzyl)pipéridine
4-(5H-dibenzo[a,d]cycloheptène-5-ylidène)-1-(4-méthybenzyl)pipéridine
4-(5H-dibenzo[a,d]cycloheptène-5-ylidène)-1-heptylpipéridine
4-(5H-dibenzo[a,d]cycloheptène-5-ylidène)-1-nonylpipéridine
4-(5H-dibenzo[a,d]cycloheptène-5-ylidène)-1-undécylpipéridine
4-(5H-dibenzo[a,d]cycloheptène-5-ylidène)-1-tridécylpipéridine
1-(2-aminobenzyl)-4-(5H-dibenzo[a,d]cycloheptène-5-ylidène)pipéridine
1-(3-aminobenzyl)-4-(5H-dibenzo[a,d]cycloheptène-5-ylidène)pipéridine
1-(4-aminobenzyl)-4-(5H-dibenzo[a,d]cycloheptène-5-ylidène)pipéridine
4-(5H-dibenzo[a,d]cycloheptène-5-ylidène)-1-(2-nitrobenzyl)pipéridine
4-(5H-dibenzo[a,d]cycloheptène-5-ylidène)-1-(3-nitrobenzyl)pipéridine
4-(5H-dibenzo[a,d]cycloheptène-5-ylidène)-1-(4-nitrobenzyl)pipéridine
4-(4-(5H-dibenzo[a,d]cycloheptène-5-ylidène)-1-pipéridinyl)-1-phényl-1-butène
1-benzyloxy-2-(4-(5H-dibenzo[a,d]cycloheptène-5-ylidène)-1-pipéridinyl)éthane
1-cyclohexyl-6-(4-(5H-dibenzo[a,d]cycloheptène-5-ylidène)-1-pipéridinyl)hexane
1-cyclohexyl-7-(4-(5H-dibenzo[a,d]cycloheptène-5-ylidène)-1-pipéridinyl)heptane
1-cyclohexyl-8-(4-(5H-dibenzo[a,d]cycloheptène-5-ylidène)-1-pipéridinyl)octane
1-(4-cyclohexylbutanoyl)-4-(5H-dibenzo[a,d]cycloheptène-5-ylidène)pipéridine
1-(2-cyanobenzyl)-4-(5H-dibenzo[a,d]cycloheptène-5-ylidène)pipéridine
1-(3-cyanobenzyl)-4-(5H-dibenzo[a,d]cycloheptène-5-ylidène)pipéridine
1-(4-cyanobenzyl)-4-(5H-dibenzo[a,d]cycloheptène-5-ylidène)pipéridine
4-(5H-dibenzo[a,d]cycloheptène-5-ylidène)-1-(2-picolyl)pipéridine
4-(5H-dibenzo[a,d]cycloheptène-5-ylidène)-1-(3-picolyl)pipéridine
4-(5H-dibenzo[a,d]cycloheptène-5-ylidène)-1-(4-picolyl)pipéridine
1-décanoyl-4-(5H-dibenzo[a,d]cycloheptène-5-ylidène)pipéridine
1-cyano-3-(4-(5H-dibenzo[a,d]cycloheptène-5-ylidène)-1-pipéridinyl)propane
1-cyano-4-(4-(5H-dibenzo[a,d]cycloheptène-5-ylidène)-1-pipéridinyl)butane
2-(4-(5H-dibenzo[a,d]cycloheptène-5-ylidène)-1-pipéridinyl)-3',4'-diméthoxyacétophénone
3-(4-(5H-dibenzo[a,d]cycloheptène-5-ylidène)-1-pipéridinyl)-3',4'-diméthoxypropiophénone
5-(4-(5H-dibenzo[a,d]cycloheptène-5-ylidène)-1-pipéridinyl)-3',4'-diméthoxyvalérophénone
4-(4-(5H-dibenzo[a,d]cycloheptène-5-ylidène)-1-pipéridinyl)-3',4',5'-triméthoxybutyrophénone
4-(4-(5H-dibenzo[a,d]cycloheptène-5-ylidène)-1-pipéridinyl)-2',3',4'-triméthoxybutyrophénone
4-(4-(5H-dibenzo[a,d]cycloheptène-5-ylidène)-1-pipéridinyl)-4'-méthoxybutyrophénone
4-(5H-dibenzo[a,d]cycloheptène-5-ylidène)-1-(2,3-diméthoxybenzyl)pipéridine
4-(5H-dibenzo[a,d]cycloheptène-5-ylidène)-1-(3,4-diméthoxybenzyl)pipéridine
4-(5H-dibenzo[a,d]cycloheptène-5-ylidène)-1-(3,4-diméthoxybenzoyl)pipéridine
1-cyclohexyl-3-(4-(5H-dibenzo[a,d]cycloheptène-5-ylidène)-1-pipéridinyl)propylcétone
2-cyclohexyl-5-(4-(5H-dibenzo[a,d]cycloheptène-5-ylidène)-1-pipéridinyl)valéronitrile
1-(4-(3-chloro-5H-dibenzo[a,d]cycloheptène-5-ylidène)-1-pipéridinyl)-4-cyclohexylbutane
1-cyclohexyl-(4-3-méthoxy-5H-dibenzo[a,d]cycloheptène-5-ylidène)-1-pipéridinyl)butane
4,9-dihydro-4-(1-(4-cylcohexylbutyl)-4-pipéridinylidène)-10H-benzo[4,5]cyclohepta[1,2-b]thiophéne-10-one
1-(4-cyclohexylbutyl)-4-(9-xantylidène)pipéridine
1-(4-cyclohexylbutyl)-4-(9-thioxantylidène)pipéridine
6,11-dihydro-11-(1-(4-cyclohexylbutyl)-4-pipéridinylidène)-5H-benzo[5,6]cyclohepta[1,2-b]pyridine
4-(10,11-dihydro-5H-dibenzo[a,d]cycloheptène-5-ylidène)-1-(3-méthoxybenzyl)pipéridine
1-décyl-4-(10,11-dihydro-5H-dibenzo[a,d]cycloheptène-5-ylidène)pipéridine
1-cyclohexyl-4-(4-(10,11-dihydro-5H-dibenzo[a,d]cycloheptène-5-ylidène)-1-pipéridinyl)butane
4-(4-(10,11-dihydro-5H-dibenzo[a,d]cycloheptène-5-ylidène)-1-pipéridinyl)-3',4'-diméthoxybutyrophénone
4-(5H-dibenzo[a,d]cycloheptène-5-ylidène)-1-(3,4,5-triméthoxybenzyl)pipéridine
ou un de leurs sels chlorhydrates ; ou l'un quelconque des composés suivants ou une de leurs bases libres :
2-(4-(5H-dibenzo[a,d]cycloheptène-5-ylidène)-1-pipéridinyl)-1-phénylbutane chlorhydrate
5-(4-(5H-dibenzo[a,d]cycloheptène-5-ylidène)-1-pipéridinyl)-1-phénylpentane chlorhydrate
6-(4-(5H-dibenzo[a,d]cycloheptène-5-ylidène)-1-pipéridinyl)-1-phénylhexane chlorhydrate
7-(4-(5H-dibenzo[a,d]cycloheptène-5-ylidène)-1-pipéridinyl)-1-phénylheptane chlorhydrate
4-(4-(5H-dibenzo[a,d]cycloheptène-5-ylidène)-1-pipéridinyl)-1-phénoxybutane chlorhydrate
2-(4-(5H-dibenzo[a,d]cycloheptène-5-ylidène)-1-pipéridinyl)-1-phénylthioéthane chlorhydrate
4-(4-(5H-dibenzo[a,d]cycloheptène-5-ylidène)-1-pipéridinyl)-1-phénylthiobutane chlorhydrate
4-(5H-dibenzo[a,d]cycloheptène-5-ylidène)-1-(2-(2-nitrobenzènesulfonylamino)éthyl)pipéridinyl) chlorhydrate
1-(2-(2-aminobenzènesulfonylamino)éthyl)-4-(5H-dibenzo[a,d]-cycloheptène-5-ylidène)pipéridine chlorhydrate
4-(5H-dibenzo[a,d]cycloheptène-5-ylidène)-1-(2-(2-éthoxycarbonylbenzènesulfonylamino)éthyl)pipéridine chlorhydrate
3-(2-(4-(5H-dibenzo[a,d]cycloheptène-5-ylidène)-1-pipéridinyl)éthyl)-2,4(1H,3H)quinazolinedione chlorhydrate
5,6-benzo-2,4-diaza(2-(4-(5H-dibenzo[a,d]cycloheptène-5-ylidène)-1-pipéridinyl)éthyl)tétrahydrothiopyrane-1,1-dioxyde chlorhydrate
5-(4-(5H-dibenzo[a,d]cycloheptène-5-ylidène)-1-pipéridinyl)-2-(3,4-diméthoxyphényl)-2-isopropylvaléronitrile chlorhydrate
4-(5H-dibenzo[a,d]cycloheptène-5-ylidène)-1-(2-(benzoylamino)éthyl)pipéridine chlorhydrate
4-(5H-dibenzo[a,d]cycloheptène-5-ylidène)-1-(2-phénylcarbamoylamino)éthyl)pipéridine chlorhydrate
5-(4-(5H-dibenzo[a,d]cycloheptène-5-ylidène)-1-pipéridinyl)-2-(3,4,5-triméthoxyphényl)-2-isopropylvaléronitrile chlorhydrate
2-(3-(4-(5H-dibenzo[a,d]cycloheptène-5-ylidène)-1-pipéridinyl)propyl)-2-phényl-1,3-dithiane-1,1,3,3-tétroxyde chlorhydrate
2-(3,4-diméthoxyphényl)-2-(3-(4-(5H-dibenzo[a,d]cycloheptène-5-ylidène)-1-pipéridinyl)-propyl)-1,3-dithiane-1,1,3,3-tétroxyde chlorhydrate
5-(4-(5H-dibenzo[a,d]cycloheptène-5-ylidène)-1-pipéridinyl)-2-(3,4-dichlorophényl)-2-isopropylvaléronitrile chlorhydrate
2-(3-benzoylphényl)-5-(4-(5H-dibenzo[a,d]cycloheptène-5-ylidène)-1-pipéridinyl)-2-méthylvaléronitrile chlorhydrate
5-(4-(5H-dibenzo[a,d]cycloheptène-5-ylidène)-1-pipéridinyl)-2,2-diphénylvaléronitrile chlorhydrate
4-(4-(5H-dibenzo[a,d]cycloheptène-5-ylidène)-1-pipéridinyl)-3,4-diméthoxybutyrophénone chlorhydrate
6-(4-(5H-dibenzo[a,d]cycloheptène-5-ylidène)-1-pipéridinyl)-2-phénylhexanenitrile chlorhydrate
6-(4-(5H-dibenzo[a,d]cycloheptène-5-ylidène)-1-pipéridinyl)-2-isopropyl-2-phénylhexanenitrile chlorhydrate
6-(4-(5H-dibenzo[a,d]cycloheptène-5-ylidène)-1-pipéridinyl)-2-(3,4-diméthoxyphényl)-2-isopropylhexanenitrile chlorhydrate
7-(4-(5H-dibenzo[a,d]cycloheptène-5-ylidène)-1-pipéridinyl)-2-phénylheptanenitrile chlorhydrate
7-(4-(5H-dibenzo[a,d]cycloheptène-5-ylidène)-1-pipéridinyl)-2-isopropyl-2-phénylheptanenitrile chlorhydrate
7-(4-(5H-dibenzo[a,d]cycloheptène-5-ylidène)-1-pipéridinyl)-2-(3,4-diméthoxyphényl)-2-isopropylheptanenitrile chlorhydrate
2-(3-chloropropyl)-5-(4-(5H-dibenzo[a,d]cycloheptène-5-ylidène)-1-pipéridinyl)-2-phénylvaléronitrile chlorhydrate
5-(4-(5H-dibenzo[a,d]cycloheptène-5-ylidène)-1-pipéridinyl)-2-phényl-2-phénylthiovaléronitrile chlorhydrate
5-(4-(5H-dibenzo[a,d]cycloheptène-5-ylidène)-1-pipéridinyl)-2-(3,4-diméthoxyphényl)-2-phénylthiovaléronitrile chlorhydrate
5-(4-(5H-dibenzo[a,d]cycloheptène-5-ylidène)-1-pipéridinyl)-2-(1-naphtyl)valéronitrile chlorhydrate
5-(4-(5H-dibenzo[a,d]cycloheptène-5-ylidène)-1-pipéridinyl)-2-(1-naphtyl)-2-isopropylvaléronitrile chlorhydrate
5-(4-(5H-dibenzo[a,d]cycloheptène-5-ylidène)-1-pipéridinyl)-2-(2-naphtyl)valéronitrile chlorhydrate
5-(4-(5H-dibenzo[a,d]cycloheptène-5-ylidène)-1-pipéridinyl)-2-(2-naphtyl)-2-isopropylvaléronitrile chlorhydrate
5-(4-(5H-dibenzo[a,d]cycloheptène-5-ylidène)-1-pipéridinyl)-2-(3-trifluorométhylphényl)valéronitrile chlorhydrate
5-(4-(5H-dibenzo[a,d]cycloheptène-5-ylidène)-1-pipéridinyl)-2-isopropyl-2-(3-trifluorométhylphényl)valéronitrile chlorhydrate
8-(4-(5H-dibenzo[a,d]cycloheptène-5-ylidène)-1-pipéridinyl)-2-phényloctanenitrile chlorhydrate
8-(4-(5H-dibenzo[a,d]cycloheptène-5-ylidène)-1-pipéridinyl)-2-isopropyl-2-phényloctanenitrile chlorhydrate
8-(4-(5H-dibenzo[a,d]cycloheptène-5-ylidène)-1-pipéridinyl)-2-(3,4-diméthoxyphényl)-2-isopropyloctanenitrile chlorhydrate
1-(3-(4-(5H-dibenzo[a,d]cycloheptène-5-ylidène)-1-pipéridinyl)propyl)-1-indanecarbonitrile chlorhydrate
1-(3-(4-(5H-dibenzo[a,d]cycloheptène-5-ylidène)-1-pipéridinyl)propyl)-5,6-diméthoxy-1-indanecarbonitrile chlorhydrate
5-(4-(5H-dibenzo[a,d]cycloheptène-5-ylidène)-1-pipéridinyl)-2-(1-méthylpyrrol-2-yl)valéronitrile chlorhydrate
5-(4-(5H-dibenzo[a,d]cycloheptène-5-ylidène)-1-pipéridinyl)-2-isopropyl-2-(1-méthylpyrrol-2-yl)valéronitrile chlorhydrate
5-(4-(5H-dibenzo[a,d]cycloheptène-5-ylidène)-1-pipéridinyl)-2-isopropyl-2-(pyrrol-2-yl)valéronitrile chlorhydrate
5-(4-(5H-dibenzo[a,d]cycloheptène-5-ylidène)-1-pipéridinyl)-2-(3-(α-hydroxybenzyl)phényl)-2-méthylvaléronitrile chlorhydrate
2-(3-benzoylphényl)-6-(4-(5H-dibenzo[a,d]cycloheptène-5-ylidène)-1-pipéridinyl)-2-méthylhexanenitrile chlorhydrate
6-(4-(5H-dibenzo[a,d]cycloheptène-5-ylidène)-1-pipéridinyl)-2-(3-(α-hydroxybenzyl)phényl)-2-méthythexanenitrile chlorhydrate
2-(3-benzoylphényl)-7-(4-(5H-dibenzo[a,d]cycloheptène-5-ylidène)-1-pipéridinyl)-2-méthylheptanenitrile chlorhydrate
7-(4-(5H-dibenzo[a,d]cycloheptène-5-ylidène)-1-pipéridinyl)-2-(3-(α hydroxybenzyl)phényl)-2-méthylheptanenitrile chlorhydrate
2-(3-benzoylphényl)-8-(4-(5H-dibenzo[a,d]cycloheptène-5-ylidène)-1-pipéridinyl)-2-méthyloctanenitrile chlorhydrate
8-(4-(5H-dibenzo[a,d]cycloheptène-5-ylidène)-1-pipéridinyl)-2-(3-(α-hydroxybenzyl)phényl)-2-méthyloctanenitrile chlorhydrate
5-(4-(5H-dibenzo[a,d]cycloheptène-5-ylidène)-1-pipéridinyl)-2-méthyl-2-phénylvaléronitrile chlorhydrate
5-(4-(5H-dibenzo[a,d]cycloheptène-5-ylidène)-1-pipéridinyl)-2-(3,4-diméthoxyphényl)-2-méthylvaléronitrile chlorhydrate
5-(4-(5H-dibenzo[a,d]cycloheptène-5-ylidène)-1-pipéridinyl)-2-éthyl-2-phénylvaléronitrile chlorhydrate
5-(4-(5H-dibenzo[a,d]cycloheptène-5-ylidène)-1-pipéridinyl)-2-(3,4-diméthoxyphényl)-2-éthylvaléronitrile chlorhydrate
5-(4-(5H-dibenzo[a,d]cycloheptène-5-ylidène)-1-pipéridinyl)-2-propyl-2-phénylvaléronitrile chlorhydrate
5-(4-(5H-dibenzo[a,d]cycloheptène-5-ylidène)-1-pipéridinyl)-2-(3,4-diméthoxyphényl)-2-propylvaléronitrile chlorhydrate
2-butyl-5-(4-(5H-dibenzo[a,d]cycloheptène-5-ylidène)-1-pipéridinyl)-2-phénylvaléronitrile chlorhydrate
2-butyl-5-(4-(5H-dibenzo[a,d]cycloheptène-5-ylidène)-1-pipéridinyl)-2-(3,4-diméthoxyphényl)valéronitrile chlorhydrate
5-(4-(5H-dibenzo[a,d]cycloheptène-5-ylidène)-1-pipéridinyl)-2-pentyl-2-phénylvaléronitrile chlorhydrate
5-(4-(5H-dibenzo[a,d]cycloheptène-5-ylidène)-1-pipéridinyl)-2-(3,4-diméthoxyphényl)-2-pentylvaléronitrile chlorhydrate
5-(4-(5H-dibenzo[a,d]cycloheptène-5-ylidène)-1-pipéridinyl)-2-hexyl-2-phénylvaléronitrile chlorhydrate
5-(4-(5H-dibenzo[a,d]cycloheptène-5-ylidène)-1-pipéridinyl)-2-(3,4-diméthoxyphényl)-2-hexylvaléronitrile chlorhydrate
5-(4-(5H-dibenzo[a,d]cycloheptène-5-ylidène)-1-pipéridinyl)-2-heptyl-2-phénylvaléronitrile chlorhydrate
5-(4-(5H-dibenzo[a,d]cycloheptène-5-ylidène)-1-pipéridinyl)-2-(3,4-diméthoxyphényl)-2-heptylvaléronitrile chlorhydrate
5-(4-(5H-dibenzo[a,d]cycloheptène-5-ylidène)-1-pipéridinyl)-2-octyl-2-phénylvaléronitrile chlorhydrate
5-(4-(5H-dibenzo[a,d]cycloheptène-5-ylidène)-1-pipéridinyl)-2-(3,4-diméthoxyphényl)-2-octylvaléronitrile chlorhydrate
5-(4-(5H-dibenzo[a,d]cycloheptène-5-ylidène)-1-pipéridinyl)-2-nonyl-2-phénylvaléronitrile chlorhydrate
5-(4-(5H-dibenzo[a,d]cycloheptène-5-ylidène)-1-pipéridinyl)-2-(3,4-diméthoxyphényl)-2-nonylvaléronitrile chlorhydrate
2-décyl-5-(4-(5H-dibenzo[a,d]cycloheptène-5-ylidène)-1-pipéridinyl)-2-phénylvaléronitrile chlorhydrate
2-décyl-5-(4-(5H-dibenzo[a,d]cycloheptène-5-ylidène)-1-pipéridinyl)-2-(3,4-diméthoxyphényl)valéronitrile chlorhydrate
4-(4-(5H-dibenzo[a,d]cycloheptène-5-ylidène)-1-pipéridinyl)-butyrophénone chlorhydrate
4-(4-(5H-dibenzo[a,d]cycloheptène-5-ylidène)-1-pipéridinyl)-1-hydroxy-1-phénylbutane chlorhydrate
5-(4-(5H-dibenzo[a,d]cycloheptène-5-ylidène)-1-pipéridinyl)-valérophénone chlorhydrate
5-(5H-dibenzo[a,d]cycloheptène-5-ylidène)-1-pipéridinyl)-1-hydroxy-1-phénylpentane chlorhydrate
4-(4-(5H-dibenzo[a,d]cycloheptène-5-ylidène)-1-pipéridinyl)-1-hydroxy-1-(4-fluorophényl)butane chlorhydrate
5-(4-(5H-dibenzo[a,d]cycloheptène-5-ylidène)-1-pipéridinyl)-4'-fluorovalérophénone chlorhydrate
5-(4-(5H-dibenzo[a,d]cycloheptène-5-ylidène)-1-pipéridinyl)-1-hydroxy-1-(4-fluorophényl)pentane chlorhydrate
5-(4-(5H-dibenzo[a,d]cycloheptène-5-ylidène)-1-pipéridinyl)-2-phénylvaleronitrile chlorhydrate
5-(4-(5H-dibenzo[a,d]cycloheptène-5-ylidène)-1-pipéridinyl)-2-isopropyl-2-phénylvaléronitrile chlorhydrate
5-(4-(5H-dibenzo[a,d]cycloheptène-5-ylidène)-1-pipéridinyl)-2-(3,4-diméthoxyphényl)valéronitrile chlorhydrate
2-(3-(4-(5H-dibenzo[a,d]cycloheptène-5-ylidène)-1-pipéridinyl)-propyl)-2-(4-fluorophényl)-1,3-dioxolane chlorhydrate
4-(4-(5H-dibenzo[a,d]cycloheptène-5-ylidène)-1-pipéridinyl)-4'-fluorobutyrophénone chlorhydrate
5-(4-(5H-dibenzo[a,d]cycloheptène-5-ylidène)-1-pipéridinyl)-2-isopropyl-2-(3,4,5-triméthoxyphényl)valéronitrile chlorhydrate
5-(4-(5H-dibenzo[a,d]cycloheptène-5-ylidène)-1-pipéridinyl)-2-(3-fluorophényl)-2-isopropylvaléronitrile chlorhydrate
5-(4-(5H-dibenzo[a,d]cycloheptène-5-ylidène)-1-pipéridinyl)-2-isopropyl-2-(3-méthoxyphényl)valéronitrile chlorhydrate
5-(4-(5H-dibenzo[a,d]cycloheptène-5-ylidène)-1-pipéridinyl)-2-isopropyl-2-(3-méthylphényl)valéronitrile chlorhydrate
5-(4-(5H-dibenzo[a,d]cycloheptène-5-ylidène)-1-pipéridinyl)-2-isopropyl-2-(2-trifluorométhylphényl)valéronitrile chlorhydrate
5-(4-(5H-dibenzo[a,d]cycloheptène-5-ylidène)-1-pipéridinyl)-2-isopropyl-2-(4-trifluorométhylphényl)valéronitrile chlorhydrate
5-(4-(5H-dibenzo[a,d]cycloheptène-5-ylidène)-1-pipéridinyl)-2-éthyl-2-(3-trifluorométhylphényl)valéronitrile chlorhydrate
2-butyl-5-(4-(5H-dibenzo[a,d]cycloheptène-5-ylidène)-1-pipéridinyl)-2-(3-trifluorométhylphényl)valéronitrile chlorhydrate
5-(4-(5H-dibenzo[a,d]cycloheptène-5-ylidène)-1-pipéridinyl)-2-hexyl-2-(3-trifluorométhylphényl)valéronitrile chlorhydrate

7. Utilisation selon l'une quelconque des revendications 1 à 6, où on utilise le dérivé de pipéridine en présence d'une substance auxiliaire, ladite substance étant un quelconque diluant inactif, liant, agent de gonflement, édulcorant, arôme, agent lubrifiant ou agent mouillant.
